(19) Europäisches Patentamt
European Patent Office
Office européen des brevets

(11) **EP 3 355 867 B1**

(12) **FASCICULE DE BREVET EUROPEEN**

(45) Date de publication et mention de la délivrance du brevet:
**18.03.2020 Bulletin 2020/12**

(21) Numéro de dépôt: **16775632.9**

(22) Date de dépôt: **27.09.2016**

(51) Int Cl.:
*A61K 9/46* (2006.01)     *A61K 9/00* (2006.01)
*A61K 9/12* (2006.01)     *A61K 31/573* (2006.01)
*A61K 31/7048* (2006.01)

(86) Numéro de dépôt international:
**PCT/EP2016/073013**

(87) Numéro de publication internationale:
**WO 2017/055296 (06.04.2017 Gazette 2017/14)**

(54) **MOUSSE CHIMIQUE NON RINCÉE COMPRENANT DE L'IVERMECTINE**

CHEMISCHER SCHAUM MIT IVERMECTIN OHNE AUSSPÜLEN

NO-RINSE CHEMICAL FOAM COMPRISING IVERMECTIN

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **29.09.2015 FR 1559206**

(43) Date de publication de la demande:
**08.08.2018 Bulletin 2018/32**

(73) Titulaire: **Galderma Research & Development**
**06410 Biot (FR)**

(72) Inventeurs:
• **BUGE, Jean-Christophe**
**06200 Nice (FR)**
• **NADAU-FOURCADE, Karine**
**06270 Villeneuve-Loubet (FR)**

(74) Mandataire: **Casalonga**
**Casalonga & Partners**
**Bayerstraße 71/73**
**80335 München (DE)**

(56) Documents cités:
**WO-A1-00/27356         FR-A1- 2 924 944**
**US-A1- 2002 061 855     US-A1- 2013 244 976**

• **DATABASE WPI Week 201535 4 mars 2015 (2015-03-04) Thomson Scientific, London, GB; AN 2015-25022D XP002753581, -& CN 104 382 863 A (ZHENGZHOU HOUYI PHARM CO LTD) 4 mars 2015 (2015-03-04)**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

**Description**

La présente invention a pour objet une composition pharmaceutique pour application topique sous forme de mousse non rincée comprenant un composé de la famille des avermectines, de préférence l'ivermectine.

La présente invention a également pour objet une composition pharmaceutique topique sous forme de mousse non rincée comprenant un composé de la famille des avermectines, de préférence l'ivermectine, pour son utilisation chez les patients présentant des pathologies cutanées inflammatoires.

La classe des avermectines est un groupe de lactones macrocycliques produit par la bactérie *Streptomyces avermitilis* (Reynolds JEF (Ed) (1993) Martindale. The extra pharmacopoeia. 29th Edition. Pharmaceutical Press, London). Parmi ces lactones macrocycliques appartenant à la classe des avermectines, on peut citer l'ivermectine, l'avermectine, l'abamectine, la doramectine, l'éprinomectine, la selamectine, l'aversectine B, AB ou C, l'émamectine B1b et leurs dérivés, ou la latidectine.

Selon l'invention, le composé de la famille des avermectines est préférentiellement l'ivermectine.

L'ivermectine est un mélange de 22,23-dihydroavermectine B1a et 22,23- dihydroavermectine B1b. L'ivermectine contient majoritairement du 22,23- dihydroavermectine B1a.

L'ivermectine est connue dans l'art antérieur pour ses propriétés antiparasitaires et anthelminthiques. Au milieu des années 80, la molécule a été présentée comme médicament antiparasitaire de large spectre à usage vétérinaire (CAMPBELL, W.C., et al., (1983). Ivermectin: a patent new antiparasitic agent. Science, 221, 823-828.). Elle est efficace contre la plupart des vers intestinaux communs (excepté les ténias), la plupart des acarides, et quelques poux. Elle présente une affinité importante pour les canaux chlorure glutamate-dépendants, notamment ceux dépendant du neuromédiateur GABA (acide gamma-amino-butyrique), présents dans les cellules nerveuses et musculaires des invertébrés, lui conférant une activité antiparasitaire. Plus particulièrement, sa fixation sur ces canaux favorise une augmentation de la perméabilité membranaire aux ions chlorures entraînant une hyperpolarisation de la cellule nerveuse ou musculaire. Il en résulte une paralysie neuromusculaire pouvant entraîner la mort de certains parasites. L'ivermectine interagit également avec d'autres canaux chlore.

L'ivermectine est utilisée classiquement dans le traitement dermatologique des manifestations endoparasitaires telles que l'onchocercose et la myase.

Les brevets US 6,133,310 et US 5,952,372 décrivent également l'utilisation de l'ivermectine dans le traitement de la rosacée afin de réduire et éliminer le parasite *Demodex folliculorum.* L'ivermectine est également connue pour son utilisation dans le traitement et/ou la prévention de bon nombre de pathologies cutanées inflammatoires telles que l'acné, l'eczéma, la dermatite atopique ou encore le psoriasis et en particulier la rosacée.

Le mécanisme d'action de l'ivermectine dans les lésions inflammatoires de la rosacée repose sur ses propriétés anti-inflammatoires et antiparasitaires. L'ivermectine élimine les acariens Demodex qui sont impliqués dans les éruptions inflammatoires de la peau. Ces acariens pouvant être présents à la fois sur la peau et le cuir chevelu, des compositions nettoyantes contenant de l'ivermectine adaptées soit au nettoyage du cuir chevelu, soit à celui de la peau ou bien des deux en même temps présentent un intérêt particulier dans cette pathologie et son traitement.

Les affections dermatologiques sont souvent liées à une sensibilité accrue de la peau, particulièrement dans le cas de la rosacée qui est une dermatose inflammatoire qui affecte principalement la partie centrale du visage et se caractérise entre autres par le rougissement du visage, des bouffées de chaleur, un érythème facial. Les patients atteints de rosacée ont une peau très sensible et, les produits nettoyants doivent présenter une très haute tolérance. Or la plupart des produits nettoyants présentent une forte teneur en tensioactifs nettoyants et moussants importante de manière à générer une quantité de mousse importante pour aider au nettoyage. Cependant les tensioactifs moussants sont généralement irritants. En outre, certains composés utilisés dans les compositions destinées à une application topique connue peuvent entraîner des effets secondaires qui peuvent en limiter l'utilisation et donc l'efficacité. Par exemple, certains actifs présentent l'inconvénient majeur d'induire de l'irritation pouvant entraîner une tolérance médiocre du produit. Cela peut ainsi créer de la part du patient un comportement de non observance du traitement et du mécontentement à l'égard dudit traitement.

Il existe donc le besoin de disposer de nouvelles formes galéniques et en particulier des compositions de type mousse ou moussantes dans lesquelles l'ivermectine soit stable, efficace et agréable à appliquer et garantissant une bonne tolérance du produit.

De façon surprenante, le Demandeur a développé des compositions pharmaceutiques moussantes, comprenant un composé de la famille des avermectines, notamment l'ivermectine, très bien tolérées par les peaux sensibles. Les mousses permettent de pallier les problèmes de tolérance par un meilleur contrôle de la dose, grâce à leurs propriétés d'étalement et leur faible densité.

La composition selon la présente invention peut être utilisée de façon intéressante chez les patients présentant des pathologies cutanées inflammatoires telles que la rosacée mais aussi l'acné, l'eczéma, ou encore la dermatite atopique.

La composition selon la présente invention, présente l'avantage d'être une émulsion sous forme de mousse générée de façon extemporanée et très bien tolérée.

Après son application, la composition selon l'invention n'est pas éliminée par rinçage.

Un des avantages de la composition est d'être particulièrement bien tolérée, malgré le fait qu'elle ne soit pas éliminée par rinçage, comme le montrent les exemples illustrant une des méthodes d'évaluation de la tolérance présentés ci-après, et au vu des figures annexées à la présente demande.

La Figure 1 montre des photographies d'une première composition conforme à l'invention obtenue par mélange des deux compositions intermédiaires A4 et B4 décrites dans les exemples, immédiatement après mélange de celles-ci puis lorsque la réaction entre ces deux compositions est complète (volume de mousse maximal).

La Figure 2 présente des photographies d'une composition de mousse selon l'invention et montre que celle-ci est stable au cours du temps comme exposé dans l'exemple 6.

Les Figures 3, 4 et 5 illustrent les résultats d'une étude comparative de profil de libération - perméation dans la peau entre une composition selon l'invention et une composition de référence, décrite dans l'exemple 5.

Il existe différentes méthodes d'évaluation de la tolérance d'un produit pharmaceutique ou cosmétique à usage cutané parmi lesquels on peut citer le test in vivo « in used » or « human patch test » mais aussi le test in vitro tel que le test de mesure de l'irritation sur Epiderme Humain Reconstruit (Reconstructed Human Epidermis ou RHE) décrit dans le protocole TG439 OCDE. Cette dernière méthode est détaillée dans l'exemple 3.

Il existe actuellement des mousses ou compositions moussantes sur le marché. Toutefois, elles présentent toutes un certain nombre d'inconvénients :

En effet, il existe 3 types de mousses ou compositions moussantes :

- Des aérosols dans lesquels la mousse est générée par un gaz propulseur mais avec l'inconvénient d'être des aérosols présentant les risques bien connus de ceux-ci (pollution, risques respiratoires notamment).
- Des crèmes foisonnées dans lesquelles des bulles d'air sont introduites dans le produit par l'intermédiaire d'un procédé de fabrication particulier. Ce procédé présente l'inconvénient d'être contraignant au niveau industriel et nécessite un lourd investissement au niveau du matériel de conditionnement.
- Des formules moussantes peu riches en tensioactifs moussants mais conditionnées dans un emballage muni d'un système mécanique générateur de mousse (pompe avec grille de type pulvorex). Ce type de formulation nécessite l'utilisation de tensioactifs moussants pouvant induire de l'irritation dans des produits non rincés.

Ainsi, il subsiste donc le besoin de mettre au point une composition pharmaceutique dont la forme galénique est différente des formes galéniques connues afin, entre autres, de disposer de compositions destinées à une application topique contenant l'ivermectine stable dans des compositions bien tolérées destinées à une application topique chez l'être humain, en particulier une application non rincée (c'est-à-dire que la composition n'est pas éliminée par rinçage après son application).

Le but de la présente invention est donc de proposer une composition répondant à ces besoins.

La demanderesse a ainsi mis au point une nouvelle composition pharmaceutique, destinée à une application topique non rincée qui se présente sous la forme d'une mousse qui ne contient avantageusement pas de tensioactif moussant. On définit par tensioactif moussant des tensioactifs qui produisent une mousse volumineuse, stable et onctueuse lorsqu'ils sont mélangés à l'eau selon les tests bien connus de l'homme du métier.

Constituent des tensioactifs moussants : les tensioactifs anioniques, les tensioactifs cationiques, les tensioactifs amphotères et les tensioactifs non ioniques de la famille des alkylpolyglucosides et des glucamides.

La forme galénique selon l'invention présente l'avantage de garantir une bonne stabilité de l'ivermectine. De plus, cette formulation conduit avantageusement à l'obtention d'une mousse douce, parfaitement tolérée et non irritante, qui permet une meilleure couverture de la zone à traiter et permet de pallier les problèmes de tolérance par un meilleur contrôle de la dose, grâce aux propriétés d'étalement et la faible densité de la mousse. De plus, l'étude de libération-pénétration décrite l'exemple 5, confirme le besoin d'obtenir une mousse qui reste en surface de la peau, afin d'agir efficacement contre le Demodex qui siège sur la peau.

Enfin, de manière avantageuse, cette forme galénique ne nécessite pas pour sa mise en œuvre l'utilisation de gaz propulseurs ou d'aérosols. Ainsi les mousses dites aérosol ou spray sont exclues du champ de l'invention. De même, les mousses de l'art antérieur de type crèmes foisonnées et/ou formules moussantes nécessitant un système mécanique générateur de mousse (type pulvorex) sont également exclues de l'invention.

La présente invention a enfin pour objet un médicament destiné à une application topique sur la peau, comprenant une telle composition.

La présente invention a également pour objet la composition selon l'invention, pour son utilisation dans le traitement et/ou la prévention des pathologies cutanées inflammatoires. De préférence, la composition selon l'invention est utilisée pour le traitement et/ou la prévention de l'acné, l'eczéma, la dermatite atopique, le psoriasis ou la rosacée et plus préférentiellement pour le traitement et/ou la prévention de la rosacée.

La présente invention sera décrite plus en détail dans la description et les exemples ci-après. La composition selon l'invention est capable de prendre la forme d'une mousse par le seul fait de sa composition et peut être également définie comme une composition auto-moussante pour application topique.

La présente invention a, en conséquence, pour premier objet une composition contenant de l'ivermectine destinée à une application topique non rincée se présentant sous la forme d'une mousse, avantageusement de consistance semi-solide, ne contenant avantageusement pas de tensioactif moussant, et comprenant un milieu pharmaceutiquement compatible avec une application topique non rincée notamment sur la peau et les phanères.

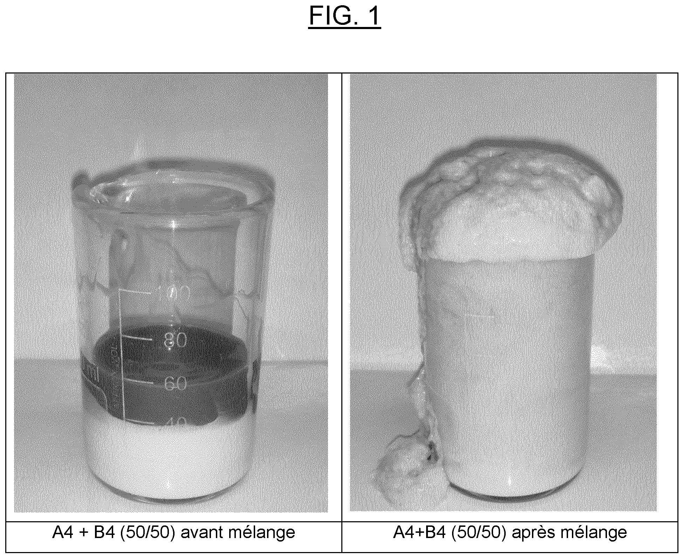

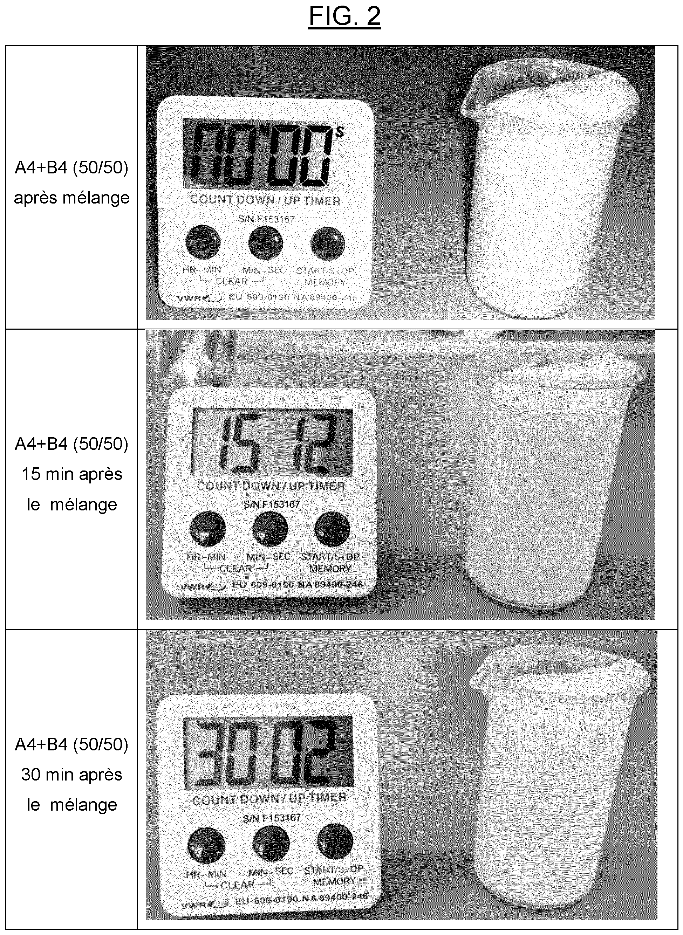

Par composition se présentant sous la forme d'une mousse, (également dénommé ci-après composition auto-moussante), il est entendu une composition de consistance semi-solide ayant une forme aérée assimilable à une mousse telle que présentée en figures 1 et 2.

La composition auto-moussante selon la présente invention comprend au moins deux compositions ou formules intermédiaires :

- au moins une composition intermédiaire B comprenant un agent générateur de gaz et au moins un agent gélifiant et/ou agent de suspensions choisi parmi les polymères d'acide acrylique, les polysaccharides la famille des aluminium magnésium silicates, la famille des amidons modifiés et la famille des carraghénanes,
- au moins une composition intermédiaire A comprenant un agent activateur de l'agent générateur de gaz et au moins un agent gélifiant et/ou agent de suspensions choisi parmi les polysaccharides, la famille des aluminium magnésium silicates, la famille des amidons modifiés, la famille des carraghénanes et le PVA, et
- de l'ivermectine contenue dans l'une au moins desdites formules intermédiaires A et B.

Selon l'invention, la composition est auto-moussante, c'est-à-dire qu'elle mousse par simple mélange des compositions intermédiaires A et B. L'invention a également pour objet la composition sous forme de mousse résultant du mélange desdites compositions intermédiaires A et B.

Selon l'invention, chaque composition (ou formule) intermédiaire peut présenter une viscosité (mesurée à 25°C et à pression atmosphérique) comprise entre 1 cP et 500000 cP, avantageusement entre 500 cP et 350000 cP, mesurée avec une méthode classique de type brookfield RV dv II : Aiguille 6 vit 2.

Selon l'invention le gaz généré par l'agent générateur de gaz peut être tout gaz physiologiquement compatible et permettant l'obtention d'une mousse, comme par exemple le dioxyde de carbone ($CO_2$) ou l'oxygène ($O_2$). De préférence, le gaz généré à partir de l'agent générateur de gaz est du dioxyde de carbone ($CO_2$).

Selon l'invention, la concentration en gaz pouvant varier, la quantité de bulles dans la composition peut varier et ainsi donner une composition pouvant aller de peu aérée à très fortement aérée.

Selon l'invention, on entend par agent activateur de l'agent générateur de gaz un ingrédient qui, par réaction chimique avec l'agent générateur de gaz, libère un gaz. Préférentiellement il s'agit d'une réaction acide/base.

Ainsi selon l'invention, la composition auto-moussante peut se présenter préférentiellement sous toutes les formes allant d'aérée à une mousse très expansée.

La composition selon l'invention est adaptée à une application topique et peut comprendre en outre un milieu physiologiquement acceptable, c'est-à-dire un milieu compatible avec la peau et les phanères. Il s'agit de préférence d'un milieu pharmaceutiquement acceptable.

En outre, la composition peut comprendre tout agent actif susceptible de présenter une activité, éventuellement thérapeutique. Ces agents actifs peuvent être, entre autres, choisis parmi les agents émollients, les agents humectants, les agents anti-radicalaires, les agents anti-inflammatoires, les vitamines, les agents dépigmentants, les agents anti-acnéiques, les agents anti-séborrhiques, les agents anti-fongiques, les agents kératolytiques, les filtres solaires, les agents amincissants, les agents de coloration de la peau.

Selon l'invention, la composition sous forme de mousse (c'est-à-dire prête à être appliquée) peut avoir un pH compris entre 2 et 8, préférentiellement entre 4 et 7.

Dans la mesure où la ou les composition(s) (ou formule(s)) intermédiaire(s) nécessite(nt) un stockage en au moins 2 compartiments pour des raisons de stabilité des ingrédients, la présente invention se rapporte soit à un unique contenant compartimenté (chaque compartiment accueillant une formule intermédiaire) et de préférence comprenant 2 ou 3 compartiments, soit à un kit comprenant chaque formule intermédiaire stockée indépendamment l'une de l'autre et physiquement séparées.

Le mélange intime extemporané (directement sur la peau ou sur tout autre support) des formules intermédiaires permet d'obtenir la composition sous forme de mousse selon l'invention.

De manière plus spécifique, la composition (ou formule) intermédiaire A peut se présenter sous forme d'une solution, d'une émulsion (lotion, crème, crème sans émulsionnant, lait, crème fluide) ou d'un gel. Cette composition contient avantageusement l'agent activateur de l'agent générateur de gaz, préférentiellement un acide en quantité suffisante (pouvant se présenter sous forme d'un tampon acide/base à pH acide) qui peut être à titre d'exemple non

4

limitatif le couple acide citrique/citrate de sodium.

La formule B peut se présenter sous forme d'une solution, d'un gel, d'une émulsion (lotion, crème, crème sans émulsionnant, lait, crème fluide). Cette composition contient avantageusement en quantité suffisante un agent générateur de gaz, qui peut être en particulier du bicarbonate de sodium.

Ainsi, l'invention a également pour objet un kit ou un contenant unique à plusieurs compartiments tel que défini précédemment, permettant la préparation extemporanée d'une composition sous forme de mousse selon l'invention, comprenant de manière séparée au moins deux formules intermédiaires (ou compositions intermédiaires) :

- une composition intermédiaire A telle que définie ci-avant comprenant au moins un agent activateur de l'agent générateur de gaz ; et
- une composition intermédiaire B telle que définie ci-avant comprenant au moins un agent générateur de gaz ;
- de l'ivermectine étant contenue dans la composition A ou dans la composition B ou simultanément dans les deux compositions A et B.

AGENT ACTIVATEUR DE GAZ :

L'agent activateur de l'agent générateur de gaz (également désigné par « agent activateur de gaz ») est un composé qui réagit avec l'agent générateur de gaz par une réaction chimique (préférentiellement une réaction acido-basique) libérant un gaz.

Il s'agit avantageusement d'un acide, d'un sel de polyacide partiellement salifié ou bien d'une solution tampon d'acide faible et de sa base conjuguée, ou d'un mélange de tels composés.

Selon l'invention le tampon acide/base dudit acide peut être tout tampon acide /base de l'acide faible comme par exemple un tampon acide citrique/citrate de sodium ou encore un tampon acide tartrique/tartrate de sodium. De préférence, nous citerons les alpha-hydroxy acides qui sont des acides faibles ayant préférentiellement un pKa compris entre 2 et 6 tel que l'acide citrique, l'acide tartrique, l'acide malique, l'acide lactique mais aussi l'acide phosphorique et l'acide pyrophosphorique et leurs sels éventuellement partiellement salifiés tels que le disodium pyrophosphate ou le sodium dihydrogénophosphate appelé encore phosphate monosodique.

Préférentiellement selon l'invention, l'agent activateur de gaz est choisi parmi un tampon acide tartrique/sel de tartrate (par exemple tartrate de sodium) ; un tampon acide citrique/citrate de sodium seul ; l'acide phosphorique, le phosphate de monosodium, le disodium pyrophosphate seuls ou en mélange avec un tampon acide citrique/ citrate de sodium.

Selon un mode de réalisation très préféré, l'agent activateur de gaz est un tampon acide citrique/ citrate de sodium seul ou en mélange avec du phosphate monosodique et/ou du disodium pyrophosphate.

Dans des compositions pour peaux sensibles ou pour peaux lésées comme les peaux acnéiques, la teneur en acide citrique/ citrate de sodium est de préférence inférieure ou égale à 2.4% par rapport au poids total de la composition intermédiaire A, de manière à limiter tout risque de picotement. Afin d'améliorer la tolérance et d'éviter la sensation de picotement, de manière préférée, le tampon acide citrique/citrate de sodium est employé en mélange avec le disodium pyrophosphate ou le sodium dihydrogénophosphate.

Selon l'invention, ledit agent activateur de gaz peut être présent dans la formule intermédiaire A en une quantité pouvant aller de 0,001% à 95% en poids par rapport au poids total de la composition intermédiaire A.

AGENT GENERATEUR DE GAZ :

Par agent générateur de gaz, on entend tout agent qui possède la propriété de générer par réaction chimique un gaz. On citera à cet égard tout composé qui, lorsqu'il est mélangé à un acide faible, peut former un gaz par une réaction chimique équivalente à la suivante :

$$NaHCO_3 + RCOOH \rightarrow RCOONa + H_2O + CO_2$$

Selon l'invention, le gaz généré à partir de l'agent générateur de gaz présent dans la composition intermédiaire B est de préférence du gaz carbonique ou dioxyde de carbone ($CO_2$), et de préférence du dioxyde de carbone ($CO_2$).

Selon l'invention, l'agent générateur de gaz est de préférence choisi parmi le bicarbonate de sodium, le bicarbonate de potassium, le carbonate de sodium, le carbonate de potassium et leurs mélanges. Préférentiellement selon l'invention, la formule intermédiaire B comprend un agent générateur de gaz carbonique qui de manière particulièrement préférée est du bicarbonate de sodium.

Ledit agent générateur de gaz peut être présent dans la formule intermédiaire B en une quantité allant de 1% à10%, préférentiellement de 2% à 8% en poids par rapport au poids de la composition intermédiaire B.

Selon l'invention, la formule intermédiaire A peut présenter un pH acide, avantageusement compris entre 1.0 et 6.0, et la formule intermédiaire B peut présenter un pH basique, avantageusement compris entre 7 et 12.

Selon l'invention, l'une (ou les) formule(s) intermédiaire (s), comprend de l'ivermectine la en quantité comprise entre 0.01% et 6% en poids par rapport au poids total de la composition totale.

De manière préférée, la composition totale (mélange de l'intermédiaire de formulation A avec l'intermédiaire de formulation B) contient de l'ivermectine à des concentrations allant de 0.5% à 5% en poids, plus préférentiellement de 0.8% à 4% en poids, par rapport au poids de la formule totale.

Dans la présente description, on entend par composition totale ou formule totale la composition du produit sous forme de mousse après le mélange desdites compositions intermédiaires. L'ivermectine est contenue dans la composition intermédiaire A ou dans la composition intermédiaire B ou simultanément dans les deux compositions.

La formule intermédiaire A, peut se présenter sous toutes les formes galéniques compatibles avec la forme galénique désirée pour la composition finale obtenue par mélange de la formule A avec la formule B. Avantageusement la formule A peut être un gel, une solution, une suspension, une émulsion (crème, crème sans tensioactif, lotion, lait, crème fluide).

La formule intermédiaire B peut se présenter sous toutes les formes galéniques compatibles avec la forme galénique désirée pour la composition finale obtenue par mélange de la formule B avec la formule A. Avantageusement la formule B peut être un gel, une solution, une suspension, une émulsion (crème, crème sans tensioactif, lotion lait, crème fluide).

Selon un mode de réalisation de l'invention, l'une des deux formules intermédiaires (ie,la formule intermédiaire A ou la formule intermédiaire B) se présente sous forme d'un gel. Dans ce mode de réalisation, de préférence l'autre formule intermédiaire n'est pas sous forme de gel.

Chaque formule intermédiaire du kit ou du contenant à plusieurs compartiments tel que défini précédemment, conforme à l'invention comprend un milieu physiologiquement acceptable, véhiculant le ou les composés, et choisi de telle sorte que les composés soient aptes à réagir l'un avec l'autre pour former une composition auto-moussante lors du mélange d'au moins les formules intermédiaires A et B.

Ainsi, le mélange extemporané d'au moins deux formules, par exemple la formule A et la formule B, crée la composition sous forme de mousse selon l'invention.

Lors du mélange des deux formules A et B, l'agent générateur de gaz tel que le bicarbonate de sodium, réagit avec l'agent activateur de gaz tel que l'acide, et donne ainsi notamment le sel correspondant à l'acide, de l'eau et le gaz $CO_2$. C'est ce gaz, emprisonné dans les bulles de la composition, qui crée la mousse qui caractérise la composition auto-moussante de l'invention.

Ainsi par le mélange d'au moins la formule intermédiaire A et la formule intermédiaire B, on obtient la composition mousse dite composition totale selon l'invention.

Dans la composition obtenue après mélange d'au moins les formules A et B, il peut bien entendu rester de l'agent activateur de gaz et/ou de l'agent générateur de gaz n'ayant pas réagi.

Avantageusement, le kit ou le contenant unique à plusieurs compartiments selon l'invention peut être conçu de telle sorte que lors de la préparation de la composition selon l'invention, les formules intermédiaires A et B peuvent être mélangées en un rapport en poids A/B allant de 0,5 à 2, préférentiellement de 0,5 à 1,5, plus préférentiellement proche de 1 (c'est-à-dire de 0,9 à 1,1), et encore plus préférentiellement de 1. Cela signifie que le kit peut être conçu pour libérer simultanément des doses (en poids) des compositions intermédiaires A et B pouvant être en un rapport en poids allant de 2 doses de B pour 1 dose de A à 2 doses de A pour 1 dose de B, de préférence de 2 doses de B pour 1 dose de A à 3 doses de A pour 2 doses de B. Selon un mode de réalisation préféré de l'invention, le kit est conçu pour libérer simultanément 1 dose en poids de A et 1 dose en poids de B.

Selon l'invention le kit peut se présenter sous toute forme compatible avec d'une part une conservation séparée des formules intermédiaires A et B et d'autre part la capacité à réaliser extemporanément le mélange de A et de B.

Par exemple les formules intermédiaires A et B peuvent être conditionnées dans un boitier avec au moins deux compartiments séparés, chacun contenant A ou B.

Selon un autre aspect, le kit peut se présenter sous la forme d'une seringue à au moins deux corps séparés, chacun muni d'un piston, lesdits deux corps contenant les formules respectives A et B, et étant conçus pour libérer simultanément par exercice d'une force sur le piston les doses désirées des formules A et B.

L'invention concerne également un procédé de préparation d'une composition sous forme de mousse selon l'invention, caractérisé en ce que pour obtenir la composition auto-moussante, on mélange de façon extemporanée une formule intermédiaire A et une formule intermédiaire B du kit telles que définies plus haut, dans des proportions relatives en poids A/B pouvant aller de 0,5 à 2, préférentiellement de 0,5 à 1,5 et plus préférentiellement de 1.

Afin d'obtenir une mousse (composition finale) optimale, les inventeurs ont cherché expérimentalement les teneurs optimales en agent générateur de gaz (de préférence le bicarbonate de sodium) et en agent activateur de gaz (de préférence l'acide citrique et/ou le disodium pyrophosphate et/ou le sodium dihydrogénophosphate ou phosphate monosodique).

Ainsi, il a été déterminé expérimentalement que lorsque l'agent activateur de gaz est l'acide citrique, le ratio en poids acide citrique/bicarbonate de sodium est avantageusement compris entre 0,1 et 2 préférentiellement entre 0,5 à 1 et de façon très préférée égal à 0,7.

De la même façon, il a été déterminé que lorsque l'agent activateur de gaz est le disodium pyrophosphate, le ratio en poids disodium pyrophosphate/bicarbonate de sodium est compris entre 0,5 et 5 préférentiellement entre 1 et 3 et de façon très préférée est égal à 2,4.

De la même façon, il a été déterminé que lorsque l'agent activateur de gaz est le sodium dihydrogénophosphate, le ratio en poids sodium dihydrogénophosphate mono hydrate/bicarbonate de sodium est compris entre 0,5 et 5 préférentiellement entre 1 et 3 et de façon très préférée est égal à 2.

L'exemplification des ratios bicarbonate de sodium/acide citrique, bicarbonate de sodium/pyrophosphate de sodium, bicarbonate de sodium/sodium hydrogénophosphate est décrit dans l'exemple 4.

De façon surprenante, l'association acide citrique/citrate de sodium, disodium pyrophosphate ou sodium dihydrogenophosphate et un système gélifiant compatible avec la forme galénique a permis d'obtenir une formule aux propriétés physico-chimiques très stables.

L'exemple 2B ci-dessous montre que les compositions selon la présente invention présentent une excellente stabilité tant physique que chimique.

Une composition est considérée stable physiquement lorsque ses caractéristiques organoleptiques, son pH, sa viscosité et l'homogénéité de l'ivermectine n'évoluent pas avec le temps dans différentes conditions de températures : température ambiante (RT, ou TA), 30°C et 40°C.

Selon l'invention, la température ambiante correspond à une température allant de 15°C à 25°C

Une composition est considérée stable chimiquement lorsque la teneur en principe actif qu'elle contient n'évolue pas avec le temps dans les différentes conditions de températures (température ambiante (RT) et 40°C).

Selon l'invention, la composition est considérée stable chimiquement quand la teneur en Ivermectine (exprimée en poids par rapport au poids de la formule intermédiaire) et mesurée par toute méthode classique et notamment HPLC, est incluse dans les spécifications allant de 90% à 110%.

La composition selon l'invention peut comprendre en outre un ou plusieurs agents choisis parmi les agents dispersants, les agents solubilisants, les agents stabilisants, les agents conservateurs, les corps gras, les agents épaississants, les colorants, les parfums, les tensioactifs, les agents gélifiants, les agents complexants, les agents neutralisants, les agents émulsionnants non moussants, les charges, les agents séquestrants, les agents réducteurs, les masques d'odeur, les agents plastifiants, les agents adoucissants, les agents hydratants, les pigments, les argiles, les charges minérales, les colloïdes minéraux, les polymères, les protéines, les agents nacrants, , les cires, les huiles comme par exemple les paraffines,les silicones, des acides gras, des esters solides d'alcool gras ou d'acides gras, des gommes, les agents mouillants.

Des colorants hydrosolubles tels que le FD&C Blue 1 (de formule brute $C_{37}H_{34}N_2Na_2O_3S_3$) et des colorants liposolubles tel que le Rouge Soudan III ou le Red Nil présentent l'avantage de colorer un des intermédiaires de formulation. Cette coloration permet le contrôle du bon mélange des deux intermédiaires de formulation et de mettre en valeur la formation de la mousse.

AGENTS GELIFIANTS DE LA FORMULE INTERMEDIAIRE CONTENANT L'ACTIVATEUR DE GAZ

La composition intermédiaire A contenant au moins un agent activateur de gaz contient au moins un agent gélifiant et/ou agent de suspension tel que défini dans la revendication 1.

La formulation A pouvant contenir de fortes quantités d'acide et d'électrolytes, elle peut s'avérer être compliquée à stabiliser. La viscosité et le pouvoir suspensif de ces formules sont souvent durs à garantir dans le temps.

A titre d'exemple de gélifiants et/ou agents de suspension résistants à la fois au aux électrolytes et aux pH acides pouvant entrer dans les compositions A selon l'invention, on peut citer les mélanges prêt à l'emploi tels que le mélange Ammonium Acrylate / Acrylamide Copolymer & Polyisobutene & Polysorbate 20 vendu par Seppic sous le nom Sepiplus 265®, le Acrylamide / Sodium Acryloyldimethyl Taurate Copolymer & Isohexadecane & Polysorbate 80 vendu par Seppic sous le nom Simulgel 600 PHA®, le Polyacrylate-13 & Polyisobutene & Polysorbate 20 vendus par Seppic sous le nom Sepiplus 400®, Acrylates/C10-30 Alkyl Acrylate Crosspolymer vendu par la société Lubrizol sous les noms Pemulen™ TR-1 Polymeric Emulsifier et Pemulen™ TR-2 Polymeric Emulsifier, les polysaccharides avec à titre d'exemples non limitatifs la gomme de xanthane telle que le Xantural180® vendu par la société Kelco, la gellan gum vendu sous le nom de Kelcogel® par la société Kelco, la Sclerotium Gum vendu sous le nom Amigel® par Alban Muller industrie, la gomme guar et ses dérivés tel que Hydroxypropyl Guar vendu sous le nom Jaguar HP-105® revendu par Rodhia, la cellulose et ses dérivés tel que la microcrystalline cellulose et carboxymethyl cellulose de sodium vendue sous le nom Blanose CMC 7H4XF® par la société Hercules, l'hydroxypropylmethylcellulose en particulier le produit vendu sous le nom de Methocel E4M® premium par la société Dow Chemical ou l'hydroxyéthylcellulose, en particulier, le produit vendu sous le nom de Natrosol HHX 250® par la société Aqualon, la famille des aluminium magnésium silicates tel que le Veegum K®, Veegum Plus® ou le Veegum Ultra® vendu par la société Vanderbilt, la bentonite vendue sous le nom de polargel HV®, la famille des amidons modifiés tels que l'amidon de pomme de terre modifié vendu sous le nom de Structure Solanace®, la famille des carraghénanes en particulier réparties sous quatre grandes familles : κ, λ, β, ω tel que les Viscarin® et les Gelcarin® commercialisés par la société IMCD. Ou encore la Polyvinyl Alcohol connue aussi sous l'abréviation PVA revendu par Merck sous le nom POLYVINYL ALCOHOL 40-88®. De façon préférée, le Vegum K®, le Simulgel 600 PHA® et le Xantural 180® seront utilisés seul ou en association deux à deux ou les trois ensemble.

L'agent gélifiant tel que décrit ci-dessus peut être utilisé aux concentrations préférentielles allant de 0,001% à 15 % et, plus préférentiellement, allant de 0,15% à 5% en poids par rapport au poids de la formule intermédiaire A.

AGENTS GELIFIANTS DE LA FORMULE INTERMEDIAIRE CONTENANT LE GENERATEUR DE GAZ

A titre d'exemple de gélifiants et/ou agent de suspension et/ou gélifiants résistants à la fois aux électrolytes et aux pH basiques pouvant entrer dans les compositions intermédiaires B selon l'invention, on peut citer les polymères d'acide acryliques comme l'Acrylates/C10-30 Alkyl Acrylate Crosspolymer tel que les carbomers dits non sensibles aux électrolytes, vendus sous le nom d'Ultrez 20®, d'Ultrez 10®, de Carbopol 1382® ou de Carbopol ETD2020NF®, AQUA SF1® vendus par la Société Lubrizol, le mélange Ammonium Acrylate / Acrylamide Copolymer & Polyisobutene & Polysorbate 20 vendu par Seppic sous le nom Sepiplus 265®, le Acrylamide / Sodium Acryloyldimethyl Taurate Copolymer & Isohexadecane & Polysorbate 80 vendu par Seppic sous le nom Simulgel 600 PHA®, le Polyacrylate-13 & Polyisobutene & Polysorbate 20 vendus par Seppic sous le nom Sepiplus 400®, Acrylates/C10-30 Alkyl Acrylate Crosspolymer vendu par la société Lubrizol sous les noms Pemulen™ TR-1 Polymeric Emulsifier et Pemulen™ TR-2 Polymeric Emulsifier, les polysaccharides avec à titre d'exemples non limitatifs la gomme de xanthane telle que le Xantural180® vendu par la société Kelco, la gellan gum vendu sous le nom de Kelcogel® par la société Kelco, la Sclerotium Gum vendu sous le nom Amigel® par Alban Muller industrie, la gomme guar et ses dérivés tel que Hydroxypropyl Guar vendu sous le nom Jaguar HP-105® revendu par Rodhia, la cellulose et ses dérivés tel que la microcrystalline cellulose et carboxymethyl cellulose de sodium vendue sous le nom Blanose CMC 7H4XF® par la société Hercules, l'hydroxypropylmethylcellulose en particulier le produit vendu sous le nom de Methocel E4M® premium par la société Dow Chemical ou l'hydroxyéthylcellulose, en particulier, le produit vendu sous le nom de Natrosol HHX 250® par la société Aqualon, la bentonite vendue sous le nom de polargel HV®, la famille des aluminium magnésium silicates tel que le Veegum K®, Veegum Plus® ou le Veegum Ultra® vendu par la société Vanderbilt, , la famille des amidons modifiés tels que l'amidon de pomme de terre modifié vendu sous le nom de Structure Solanace® ou de Farine de Tapioca connue sous le nom de Naviance Tapioca P® revendu par Akzo Novel ou de la famille des carraghénanes en particulier réparties sous quatre grandes familles : κ, λ, β, ω tel que les Viscarin® et les Gelcarin® commercialisés par la société IMCD. De façon préférée, le Vegum K®, le Simulgel 600 PHA® et le Xantural 180® seront utilisés seul ou en association deux à deux ou les trois ensemble. L'agent gélifiant tel que décrit ci-dessus peut être utilisé aux concentrations préférentielles allant de 0,001% à 15 % et, plus préférentiellement, allant de 0,15% à 5% en poids par rapport au poids de la formule intermédiaire B.

AGENTS HUMECTANTS

Parmi les agents humectants et/ou émollients qui ont pour rôle d'hydrater la peau et de faciliter l'application de la formulation, on utilise optionnellement, sans que cette liste soit limitative, des composés tels qu'un polyol miscible à l'eau à la température ambiante (25 °C) notamment choisi parmi les polyols ayant notamment de 2 à 20 atomes de carbones, de préférence ayant de 2 à 10 atomes de carbone, et préférentiellement ayant de 2 à 6 atomes de carbones, tels que le glycérol, les dérivés de glycol tel que le propylène glycol, le butylène glycol, le pentylène glycol, l'hexylène glycol, le dipropylène glycol, le diéthylène glycol, le propylene 1.3 glycol vendu sous le nom de Zemea par la société DuPont Tate & Lyle Bio Products Company, LLC et leurs mélanges mais aussi les sucres (à titre d'exemple le glucose, le lactose), les polyethylene glycol (PEG) (à titre d'exemple le Lutrol E400®), l'urée, les acides aminés (à titre d'exemple la sérine, la citrulline, l'arginine, l'asparagine, l'alanine).

A titre d'agent humectant et/ou émollient préféré, on peut citer la glycérine et le propylène glycol.

Les agents humectants peuvent être utilisés, seuls ou en association, aux concentrations préférentielles allant de 0,001% à 30 % et, plus préférentiellement, allant de 0.01% à 10% en poids par rapport au poids de la formule totale.

AGENTS CHELATANTS

Parmi les agents chélatants, on peut citer à titre d'exemples non limitatifs l'acide éthylène diamine tétra-acétique (EDTA), l'acide diéthylène triamine penta-acétique (DTPA), l'acide éthylène diamine-di (O-hydroxyphényl acétique) (EDDHA), l'acide hydroxy-2-éthylène diamine triacétique (HEDTA), l'acide éthyldiamine-di (O-hydroxy-p-méthyl phényl) acétique (EDDHMA) et l'acide éthylène diamine-di (5-carboxy-2-hydroxyphényl) acétique (EDDCHA). A titre d'agent chélatant préféré, on peut citer l'acide éthylène diamine tétra-acétique (EDTA) vendu notamment sous le nom Titriplex III® il peut être utilisé aux concentrations préférentielles allant de 0,001% à 1 % et, plus préférentiellement de 0.05% à 0.1% en poids par rapport au poids de la formule totale.

## EXCIPIENTS OU ACTIFS AUX PROPRIETES COMPLEMENTAIRES

La composition selon l'invention peut contenir un ou plusieurs excipients aux propriétés spécifiques comme par exemple, à titre non limitatif, l'allantoine aux propriétés anti-irritantes, le dipotassium glycyrrhizate pour ces propriétés anti-inflammatoires ou encore l'alpha bisabolol cicatrisant, le lithium digluconate pour ses propriétés anti-rougeurs.

Dans un mode particulier, la composition selon l'invention peut également contenir un ou plusieurs autres principes actifs pharmaceutiques comme par exemple la brimonidine ou encore un anti-inflammatoire non stéroïdien comme le diclofénac ou bien encore l'adapalène.

## CHARGES ET PARTICULES

Des charges et/ou des particules peuvent être utilisées pour stabiliser et booster la mousse. Certaines d'entre elles ont la propriété particulière de se placer à l'interface eau/air et ainsi stabiliser cette interface. Comme charges, on peut citer le talc, les oxydes de métaux tel que l'oxyde de zinc, le dioxyde de titane TiO2 T2000 vendu par la société Merck sous le nom Eusolex® T-2000, les argiles tel que les laponites, bentones ou les bentonites mais aussi les ethers de cellulose tel que le Methocel® K100 LV commercialisé par la société DOW, les silices telles que l'Aerosil® R972 vendue par la société EVONIK ou la SILICE HDK® H13L vendue par WACKER elles peuvent être utilisées aux concentrations allant de 0.01% à 10% en poids par rapport au poids de la formule totale.

## LES HUILES DE LA PHASE GRASSE

La composition selon l'invention peut également comprendre une phase grasse. Cette phase grasse peut être présente dans l'une et/ou l'autre des compositions intermédiaires A et B. La phase grasse peut selon la forme galénique des formules intermédiaires représenter de 0% à 95% en poids par rapport au poids de chaque formule intermédiaire.

La phase grasse de la composition selon l'invention peut comprendre par exemple, les huiles végétales, minérales, animales ou synthétiques, des huiles de silicones, et leurs mélanges.

Comme exemple d'huile minérale, on peut citer par exemple des huiles de paraffine de différentes viscosités telles que le Primol 352®, le Marcol 82®, Marcol 152® vendus par la société Esso.

Comme huile végétale, on peut citer l'huile d'amande douce, l'huile de palme, l'huile de soja, l'huile de sésame, l'huile de tournesol, l'huile d'olive, l'huile de sésame.

Comme huile animale ou leur substitut d'origine végétale, on peut citer la lanoline, le squalene, l'huile de poisson, avec comme dérivé le perhydrosqualene vendu sous le nom Sophiderm® par la société Sophim.

Comme huile synthétique, on peut citer un ester tel que le cetearyl isononanoate comme le produit vendu sous le nom de Cetiol SN PH® par la société Cognis France, l'isononyl isononanoate comme le DUB ININ® revendu par les Stearine Dubois, l'isopropyl myristate vendu sous le nom de crodamol IPM par la société CRODA, le diisopropyl adipate comme le produit vendu sous le nom de Crodamol DA® par la société Croda, le palmitate d'isopropyle comme le produit vendu sous le nom de Crodamol IPP® par la société Croda, le caprylique caprique triglyceride tel que Miglyol 812® vendu par la société Univar. Comme polyisobutene hydrogéné, on peut citer les Parleam® vendus par la société Rossow, le C12-15 alkyl benzoate vendu sous le nom CRODAMOL AB par la société CRODA, l'octyldodecanol ou Eutanol G vendu par la société BASF, l'alcool oléique vendu sous le nom de KOLLICREAM OA par la société BASF, le PPG-11 STEARYL ETHER l'Arlamol PS11E vendu par la société CRODA.

Comme huile de silicone, on peut citer une dimethicone comme le produit vendu sous le nom de Q7-9120 Silicone Fluid® de viscosité de 20 cst à 12500 cst par la société Dow Corning, une cyclomethicone comme le produit vendu sous le nom de ST-Cyclomethicone 5NF® également par la société Dow Corning.

A titre d'huile préférée, on peut citer le Caprylique Caprique Triglyceride, le PPG-11 Stearyl Ether ou encore l'Isopropyl Palmitate.

Ces huiles peuvent être présentes, seules ou en association, à des teneurs allant de 0.5 à 50% en poids et préférentiellement de 2 à 30% en poids, par rapport au poids de la composition totale.

## LES CORPS GRAS NON LIQUIDES

La composition selon l'invention, et en particulier la formule intermédiaire B, peut également comprendre des corps gras solides tel que des cires naturelles ou synthétiques, des acides gras tels que l'acide stéarique, des alcools gras tels que le Speziol C18® pharma ou le Speziol C16® vendu par la société Cognis, et des agents de texture de type tribehenate, tel que le Compritol 888® vendu par la société Gattefossé ou les huiles de ricin hydrogénées telles que le Cutina HR® vendu par la société Cognis ou le glyceryl stearate tel que le GELEOL® vendu par la société Gattefosse ou encore le Dc 9045 Elastomer Blend® vendu par la société Dow Corning.

Ces corps gras non liquides peuvent être utilisés seuls ou en mélange de 0 à 30% en poids par rapport au poids de la formule totale. Il a été cependant observé une qualité de mousse exceptionnelle lorsque des alcool gras de formule CH3(CH2)nOH (n est compris entre 11 et 23) sont présents à des teneurs supérieures à 1% en poids par rapport au poids de la formule totale.

EMULSIFIANTS NON IONIQUES

La composition selon l'invention et notamment la formule intermédiaire B peut également comprendre un ou plusieurs émulsifiants non ioniques.

A titre d'émulsifiants préférés on peut citer des émulsifiants hydrophiles de type Glyceryl Stearate (and) PEG-100 Stearate vendu sous le nom Arlacel 165FL® par la société Uniquema, des émulsifiants lipophiles de type Glucate SS® et Glucamate SSE®, Polyoxyethylene (21) Stearyl Ether vendu sous le nom Brij721® par la société Uniquema ou encore dans la meme famille le Brij S2®, le Brij S20®. La self emulsifying Wax vendu par Croda sous le nom de Polawax NF®. On peut citer également des tensioactifs non ioniques présentant une HLB élevée, les esters de sorbitan tels que le POE(20) sorbitan monooléate, vendu sous le nom de « Tween 80® » (HLB=15) ; le POE(20) sorbitan monostéarate vendu sous le nom de « Tween 60® » (HLB=14.9) ; les éthers d'alcools gras tels que le POE (21) stéaryl ether (HLB= 15.5), ou le ceteareth 20 vendu sous le nom de Eumulgin B2 PH® par Cognis (HLB de 15,5, ou des tensioactifs non ioniques de bas HLB, les esters de sorbitan, tels que le monostéarate de sorbitan (vendu sous le nom de Span 60® par Uniquema), les esters de glycérol tels que le monostéarate de glycérol (Cutina GMS® de chez Cognis), les esters de saccharose de bas HLB comme le distéarate de saccharose. Dans un autre mode selon l'invention les tensioactifs utilisables sont les esters de polyglycerol. Il s'agit d'esters d'acides gras polyglycerinés obtenus par condensation de glycerine. Des émulsionnants glycolipidique tel que le Montanov 202® vendu par la société SEPPIC. Certains émulsionnants peuvent être vendus sous forme de mélange tel que les Emulium Kappa® et Emulium Delta® vendu par Gattefosse. Ces Tensio actifs peuvent être utilisés seuls ou en association de façon à ce que le HLB du système soit supérieur à 11 et préférentiellement supérieur à 15.

De tels émulsifiants peuvent être utilisés entre 0.01% et 30 % en poids par rapport au poids de la composition totale, préférentiellement entre 0.1% et 15%, et plus préférentiellement entre 0.5% et 7 %.

Dans un mode préféré, l'émulsionnant est le Cetareth-20 seul ou en mélange et notamment avec le monostearate de Sorbitan.

AGENTS CONSERVATEURS :

On peut citer à titre d'exemple de conservateurs, le chlorure de benzalkonium, le bronopol, la chlorhexidine, le chlorocrésol et ses dérivés, l'alcool éthylique, le phénoxyéthanol, le sorbate de potassium, la diazolidinylurée, l'alcool benzylique, les parabens, le benzoate de sodium ou leurs mélanges.

Au titre de système conservateur préféré on peut citer le phenoxyethanol seul ou en mélange avec tout autre agent conservateur et en particulier avec ceux cités précédemment.

Les exemples suivants illustrent l'invention sans en restreindre la portée.

EXEMPLES

EXEMPLE 1 : exemples de formules

Exemples de Formules A : Compositions intermédiaires A contenant l'agent activateur de gaz:

Les formules A1, A7 et A10 ont été réalisées suivant le protocole suivant :

Etape 1 : A une température supérieure à 60°C, Ajouter sous agitation le gélifiant principal (Veegum K) à la phase d'eau principale.
Etape 2 : Ajouter les agents tampons acide puis refroidir à 40°C
Etape 3 : Ajouter le/les autre(s) gélifiant(s) sous agitation
Etape 4 : Ajouter les autres excipients de la formule sous agitation

La formule A2 a été réalisée suivant le protocole suivant :

Etape 1 : A une température de 75°C, disperser l'agent gélifiant et solubiliser les excipients hydrosolubles.

Etape 2 : Parallèlement, chauffer la phase grasse (tensioactifs, cires et huiles) + le mélange propylène glycol/ alcool oléique/ivermectine à 75°C.

Etape 3 : A 75°C, réaliser l'émulsion

Etape 4 : Ajouter les additifs tels que les conservateurs à une température adaptée à l'additif.

Etape 5 : Neutraliser le mélange.

Etape 6 : Ajouter les agents tampons acide.

Les formules A3, A4, A5, A6, A8 et A9 sont réalisées suivant le protocole de la formule A2.

Dans les exemples de formules ci-dessous, les quantités sont exprimées par rapport au poids de la formule intermédiaire et non par rapport au poids de la formule totale.

Exemple A1

| INCI Name | % |
| --- | --- |
| EAU | QSP 100 |
| DISODIUM EDTA | 0.1 |
| MAGNESIUM ALUMINIUM SILICATE | 2.5 |
| XANTHAN GUM | 1.5 |
| CITRIC ACID | 1.4 |
| SODIUM CITRATE | 1 |
| DISODIUM PYROPHOSPHATE | 7.2 |
| POLOXAMER 124 | 0.2 |
| PROPYLENE GLYCOL | 4 |
| SODIUM BENZOATE | 0.2 |

Exemple A2

| INCI Name | % |
| --- | --- |
| EAU | QSP100 |
| XANTHAN GUM | 0.5 |
| MAGNESIUM ALUMINIUM SILICATE | 2.5 |
| CETOSTEARYL ALCOHOL | 3 |
| CETEARETH-20 | 3 |
| GLYCERYL DIBEHENATE | 3 |
| CAPRYLIC/CAPRIC TRIGLYCERIDE | 6 |
| CITRIC ACID | 1.5 |
| SODIUM CITRATE | 0.5 |
| SODIUM DIHYDRONEPHOSPHATE | 6.2 |
| OLEYL ALCOHOL | 2 |

(suite)

| INCI Name | % |
|---|---|
| PROPYLENE GLYCOL | 2 |
| PHENOXYETHANOL | 0.8 |
| IVERMECTIN | 2 |

Exemple A3 :

| INCI Name | % |
|---|---|
| EAU | QSP100 |
| XANTHAN GUM | 0.5 |
| ACRYLAMIDE,AMPS COPOLYMER DISPERSION 40%/ISOHEXADECANE/POLYSORBATE 80 | 1.5 |
| CETOSTEARYL ALCOHOL | 3 |
| CETEARETH-20 | 3 |
| GLYCERYL DIBEHENATE | 3 |
| CAPRYLIC/CAPRIC TRIGLYCERIDE | 6 |
| CITRIC ACID | 1.5 |
| SODIUM CITRATE | 0.5 |
| SODIUM DIHYDRONEPHOSPHATE | 6.2 |
| OLEYL ALCOHOL | 2 |
| PROPYLENE GLYCOL | 2 |
| PHENOXYETHANOL | 0.8 |
| IVERMECTIN | 2 |

Exemple A4

| INCI Name | % |
|---|---|
| EAU | QSP100 |
| DISODIUM EDTA | 0.1 |
| XANTHAN GUM | 0.5 |
| ACRYLAMIDE,AMPS COPOLYMER DISPERSION 40%/ISOHEXADECANE/POLYSORBATE 80 | 1.5 |
| CETOSTEARYL ALCOHOL | 3 |
| CETEARETH-20 | 3 |
| GLYCERYL DIBEHENATE | 3 |
| CYCLOPENTASILOXANE | 2 |
| PPG-11 STEARYL ETHER | 5 |
| CITRIC ACID | 1.5 |
| SODIUM CITRATE | 0.5 |

(suite)

| INCI Name | % |
|---|---|
| SODIUM DIHYDRONEPHOSPHATE | 6.2 |
| OLEYL ALCOHOL | 2 |
| PROPYLENE GLYCOL | 2 |
| PHENOXYETHANOL | 1 |
| IVERMECTIN | 2 |

Exemple A5

| INCI Name | % |
|---|---|
| EAU | QSP100 |
| XANTHAN GUM | 0.5 |
| MAGNESIUM ALUMINIUM SILICATE | 2.5 |
| CETOSTEARYL ALCOHOL | 3 |
| CETEARETH-20 | 3 |
| GLYCERYL DIBEHENATE | 3 |
| CAPRYLIC/CAPRIC TRIGLYCERIDE | 6 |
| CITRIC ACID | 1.5 |
| SODIUM CITRATE | 0.5 |
| SODIUM DIHYDRONEPHOSPHATE | 6.2 |
| OLEYL ALCOHOL | 2 |
| PROPYLENE GLYCOL | 2 |
| PHENOXYETHANOL | 0.8 |

Exemple A6

| INCI Name | % |
|---|---|
| EAU | QSP 100 |
| XANTHAN GUM | 0.5 |
| MAGNESIUM ALUMINIUM SILICATE | 1 |
| ISOPROPYL PALMITATE | 4 |
| IVERMECTIN | 2 |
| CETYL ALCOHOL | 3.5 |
| STEARYL ALCOHOL | 2.5 |
| SORBITAN MONOSTEARATE | 2 |
| PROPYL PARABEN | 0.1 |
| DIMETHICONE 20 CST | 0.5 |

(suite)

| INCI Name | % |
|---|---|
| CETEARETH 20 | 3 |
| GLYCEROL | 4 |
| METHYL PARABEN | 0.2 |
| DISODIUM EDTA | 0.05 |
| CITRIC ACID | 1.5 |
| SODIUM CITRATE | 0.5 |
| SODIUM DIHYDRONEPHOSPHATE | 6.2 |
| OLEYL ALCOHOL | 2 |
| PROPYLENE GLYCOL | 2 |
| PHENOXYETHANOL | 1 |

Exemple A7

| INCI Name | % |
|---|---|
| EAU | QSP 100 |
| DISODIUM EDTA | 0.1 |
| XANTHAN GUM | 0,7 |
| MAGNESIUM ALUMINIUM SILICATE | 2.5 |
| SODIUM BENZOATE | 0.2 |
| DISODIUM PYROPHOSPHATE | 7.2 |
| CITRIC ACID | 1.4 |
| SODIUM CITRATE | 1 |
| POLOXAMER 124 | 0,2 |
| IVERMECTIN | 2 |
| PROPYLENE GLYCOL | 4,0 |

Exemple A8

| INCI Name | % |
|---|---|
| EAU | QSP 100 |
| XANTHAN GUM | 0.5 |
| ACRYLAMIDE,AMPS COPOLYMER DISPERSION 40%/ISOHEXADECANE/POLYSORBATE 80 | 1.5 |
| ISOPROPYL PALMITATE | 4 |
| IVERMECTIN | 2 |
| CETYL ALCOHOL | 3.5 |
| STEARYL ALCOHOL | 2.5 |

(suite)

| INCI Name | % |
|---|---|
| SORBITAN MONOSTEARATE | 2 |
| PROPYL PARABEN | 0.1 |
| DIMETHICONE 20 CST | 0.5 |
| CETEARETH 20 | 3 |
| GLYCEROL | 4 |
| METHYL PARABEN | 0.2 |
| DISODIUM EDTA | 0.05 |
| CITRIC ACID | 1.5 |
| SODIUM CITRATE | 0.5 |
| SODIUM DIHYDRONEPHOSPHATE | 6.2 |
| OLEYL ALCOHOL | 2 |
| PROPYLENE GLYCOL | 2 |
| PHENOXYETHANOL | 1 |

Exemple A9

| INCI Name | % |
|---|---|
| EAU | QSP 100 |
| XANTHAN GUM | 0.5 |
| ACRYLAMIDE,AMPS COPOLYMER DISPERSION 40%/ISOHEXADECANE/POLYSORBATE 80 | 1.5 |
| CAPRYLIC/CAPRIC TRIGLYCERIDE | 4.5 |
| IVERMECTIN | 2 |
| CETYL ALCOHOL | 3.5 |
| STEARYL ALCOHOL | 2.5 |
| SORBITAN MONOSTEARATE | 2 |
| PROPYL PARABEN | 0.1 |
| CETEARETH 20 | 3 |
| GLYCEROL | 4 |
| METHYL PARABEN | 0.2 |
| DISODIUM EDTA | 0.05 |
| CITRIC ACID | 1.5 |
| SODIUM CITRATE | 0.5 |
| SODIUM DIHYDRONEPHOSPHATE | 6.2 |
| OLEYL ALCOHOL | 2 |
| PROPYLENE GLYCOL | 2 |
| PHENOXYETHANOL | 1 |

Exemple A10

| INCI Name | % |
|---|---|
| EAU | QSP 100 |
| XANTHAN GUM | 0.5 |
| MAGNESIUM ALUMINIUM SILICATE | 2.5 |
| GLYCEROL | 6 |
| DISODIUM EDTA | 0.5 |
| CITRIC ACID | 1.5 |
| SODIUM CITRATE | 0.5 |
| SODIUM DIHYDRONEPHOSPHATE | 6.2 |
| METHYL PARABEN | 0.2 |
| IVERMECTIN | 2 |

Exemples de Formules B : Compositions intermédiaires B contenant l'agent générateur de gaz:

Les formules intermédiaires B sont préparées suivant le procédé suivant :

Etape 1' : A une température supérieure à 60°C, Ajouter sous agitation les gélifiants à la phase d'eau principale.
Etape 2' optionnel : Parallèlement chauffer la phase grasse (contenant les huiles, les cires, et les tensio-actifs) à une température supérieure à 60°C et incorporer l'Ivermectine.
Etape 3' I : A une température supérieure à 60°C réaliser l'émulsion en ajoutant la phase grasse à la phase principale.
Etape 4' : Ajouter les additifs tels que les conservateurs ou de l'éthanol à une température adaptée à l'additif.
Etape 5' : Neutraliser le mélange.
Etape 6' : A une température inférieure à 40°C ajouter le bicarbonate de sodium

Exemple B1

| INCI Name | % |
|---|---|
| EAU | QSP 100 |
| MAGNESIUM ALUMINIUM SILICATE | 2.5 |
| XANTHAN GUM | 0.6 |
| POLYSORBATE 80 | 0.8 |
| STEARETH-20 | 2.8 |
| CETOSTEARYL ALCOHOL | 1.5 |
| MINERAL OIL | 8 |
| TRIETHANOLAMINE | 1.2 |
| SODIUM HYDROGENO CARBONATE | 5 |

Exemples B2 et B3

| INCI NAME | B2 % | B3 % |
|---|---|---|
| EAU | QSP 100 | QSP 100 |
| SODIUM CARBOXYMETHYLCELLULOSE | 0.5 | 0.2 |
| HYDROXYETHYLCELLULOSE | 1 | 0.1 |
| STEARETH-20 | 1.8 | 1.8 |
| GLYCERYL STEARATE (AND) PEG-100 STEARATE | 2.7 | 2.7 |
| CETOSTEARYL ALCOHOL | 1 | 7 |
| HYDROGENATED POLYISOBUTENE | 9 | 9 |
| TRIETHANOLAMINE | 1.2 | 1.2 |
| PROPYLENE GLYCOL | 5 | 5 |
| PHENOXYETHANOL | 1 | 1 |
| SODIUM HYDROGENO CARBONATE | 3 | 3 |

Exemple B4

| INCI Name | % |
|---|---|
| EAU | QSP 100 |
| MAGNESIUM ALUMINIUM SILICATE | 2.5 |
| XANTHAN GUM | 0.7 |
| DISODIUM EDTA | 0.1 |
| SODIUM HYDROXYDE | 0.019 |
| SODIUM HYDROGENO CARBONATE | 5 |
| PHENOXYETHANOL | 8 |
| BENZYL ALCOHOL | 0.2 |
| FD&C BLUE 1 | 0.005 |

Exemple B5

| INCI Name | % |
|---|---|
| EAU | QSP 100 |
| MAGNESIUM ALUMINIUM SILICATE | 3.5 |
| XANTHAN GUM | 1 |
| POLYSORBATE 80 | 2 |
| STEARIC ACID | 3 |
| CETOSTEARYL ALCOHOL | 1.5 |
| HYDROGENATED POLYISOBUTENE | 8 |
| TRIETHANOLAMINE | 1.8 |
| SODIUM HYDROGENOCARBONATE | 3 |

(suite)

| INCI Name | % |
|---|---|
| PHENOXYETHANOL | 0.8 |

Exemple B6

| INCI Name | % |
|---|---|
| EAU | QSP100 |
| DISODIUM EDTA | 0.1 |
| XANTHAN GUM | 0.5 |
| ACRYLAMIDE,AMPS COPOLYMER DISPERSION 40%/ ISOHEXADECANE/ POLYSORBATE 80 | 1.5 |
| CETOSTEARYL ALCOHOL | 3 |
| CETEARETH-20 | 3 |
| GLYCERYL DIBEHENATE | 3 |
| CYCLOPENTASILOXANE | 2 |
| PPG-11 STEARYL ETHER | 5 |
| SODIUM HYDROGENOCARBONATE | 5 |
| OLEYL ALCOHOL | 2 |
| PROPYLENE GLYCOL | 2 |
| PHENOXYETHANOL | 1 |
| SODIUM HYDROXYDE | QSpH |
| IVERMECTIN | 2 |

Exemple B7

| INCI Name | % |
|---|---|
| EAU | QSP 100 |
| MAGNESIUM ALUMINIUM SILICATE | 2.5 |
| XANTHAN GUM | 0.5 |
| CETEARETH-20 | 3 |
| CETOSTEARYL ALCOHOL | 3 |
| GLYCERYL DIBEHENATE | 3 |
| CAPRYLIC/CAPRIC TRIGLYCERIDE | 6 |
| SODIUM HYDROXYDE | 0.09 |
| PHENOXYETHANOL | 0.8 |
| SODIUM HYDROGENOCARBONATE | 5 |

Exemple B8

| INCI Name | % |
|---|---|
| EAU | Qsp100 |
| ISOPROPYL PALMITATE | 4 |
| CETYL ALCOHOL | 3.5 |
| STEARYL ALCOHOL | 2.5 |
| CETEARETH 20 | 3 |
| SORBITAN MONOSTEARATE | 2 |
| DIMETHICONE 20 CST | 2 |
| PROPYL PARABEN | 0.1 |
| GLYCEROL | 4 |
| XANTHAN GUM | 0.5 |
| MAGNESIUM ALUMINIUM SILICATE | 1 |
| METHYL PARABEN | 0.2 |
| DISODIUM EDTA | 0.05 |
| SODIUM HYDROGENOCARBONATE | 5 |
| OLEYL ALCOHOL | 2 |
| PROPYLENE GLYCOL | 2 |
| PHENOXYETHANOL | 1 |
| SODIUM HYDROXYDE | QS pH |
| IVERMECTINE | 2 |

Exemple B9

| INCI Name | % |
|---|---|
| EAU | QSP100 |
| DISODIUM EDTA | 0.1 |
| XANTHAN GUM | 0.5 |
| ACRYLAMIDE,AMPS COPOLYMER DISPERSION 40%/ ISOHEXADECANE/ POLYSORBATE 80 | 1.5 |
| CETOSTEARYL ALCOHOL | 3 |
| CETEARETH-20 | 3 |
| GLYCERYL DIBEHENATE | 3 |
| CYCLOPENTASILOXANE | 2 |
| PPG-11 STEARYL ETHER | 5 |
| SODIUM HYDROGENOCARBONATE | 5 |
| OLEYL ALCOHOL | 2 |
| PROPYLENE GLYCOL | 2 |

(suite)

| INCI Name | % |
|---|---|
| PHENOXYETHANOL | 1 |
| SODIUM HYDROXYDE | QSpH |

Exemple B10

| INCI Name | % |
|---|---|
| EAU | QSP100 |
| SODIUM HYDROXYDE | QSpH |
| XANTHAN GUM | 0.5 |
| MAGNESIUM ALUMINIUM SILICATE | 2.5 |
| CETOSTEARYL ALCOHOL | 3 |
| CETEARETH-20 | 3 |
| GLYCERYL DIBEHENATE | 3 |
| CAPRYLIC/CAPRIC TRIGLYCERIDE | 6 |
| SODIUM HYDROGENOCARBONATE | 5 |
| OLEYL ALCOHOL | 2 |
| PROPYLENE GLYCOL | 2 |
| PHENOXYETHANOL | 0.8 |
| IVERMECTIN | 2 |

Le tableau ci-dessous représente les mélanges dans un ratio pondéral 1:1 des compositions intermédiaires A et B décrites ci-avant. Une croix à l'intersection de deux intermédiaires de formulation indique que le mélange a été testé et a généré une mousse aux propriétés recherchées.

| Formule B / Formule A | B1 | B2 | B3 | B4 | B5 | B6 | B7 | B8 | B9 | B10 |
|---|---|---|---|---|---|---|---|---|---|---|
| A1 | | | | | | X | | X | | X |
| A2 | X | X | X | X | X | X | X | X | X | X |
| A3 | X | X | X | X | X | X | X | X | X | X |
| A4 | X | X | X | X | X | X | X | X | X | X |
| A5 | | | | | | X | | X | | X |
| A6 | X | X | X | X | X | X | X | X | X | X |
| A7 | X | X | X | | X | X | X | X | X | X |
| A8 | X | X | X | X | X | X | X | X | X | X |
| A9 | X | X | X | X | X | X | X | X | X | X |
| A10 | X | X | X | | X | X | X | X | X | X |

EXEMPLE 2A : mesures de densité de mousse

A partir des exemples de formules décrites dans l'exemple 1, les formules A pour la formule acide et B pour la formule basique (contenant le générateur de gaz ; de préférence le bicarbonate de sodium), des mesures de densité sont réalisées sur chacune des 2 formules intermédiaires A et B (T0) puis les mesures sont réalisées sur la mousse obtenue par mélange de ces deux intermédiaires

Densité formule A4 = 1.052
Densité formule B4= 1.042
Densité de la mousse A4 /B4 (50/50)= 0.316

La mesure de la densité de la mousse montre que le volume a été augmenté d'un facteur 3.3 et confirmé par les photos en Figure 1. La photo de gauche représente le moment du mélange (T0) et la photo de droite représente la mousse obtenue lorsque la réaction chimique acide/ base est complète

EXEMPLE 2B : stabilité

Les tableaux Ia, Ib, Ic, Id, Ie et If ci-dessous rassemblent les données de stabilité physique des formules intermédiaires A2, A4, A6, A8, A9 et A10 décrites dans l'exemple 1, contenant de l'ivermectine.

Tableau Ia :

| Formule A 2 | T0 | | T1Mois | T3Mois | T3Mois |
|---|---|---|---|---|---|
| Aspect macroscopique | Emulsion blanche, lisse, opaque et épaisse | TA | Conforme | Conforme | Conforme |
| | | 40°C | Conforme | Conforme | Conforme |
| Observations microscopiques X400 | Emulsion gouttelettes d'huile inferieures 4$\mu$m | TA | Conforme | Conforme | Conforme |
| | | 40°C | Conforme | Conforme | Conforme |
| brookfield RV DVII Aig5vit5 Viscosité cP | 570400 | TA | 57840 | 66000 | 65600 |
| | | 40°C | - | - | 77500 |

(suite)

| Formule A 2 | T0 | | T1Mois | T3Mois | T3Mois |
|---|---|---|---|---|---|
| pH | 3.42 | TA | 3.54 | 3.61 | 3.61 |
| | | 40°C | 3.73 | 3.92 | 3.84 |

Tableau Ib :

| Formule A 4 | T0 | | T1Mois | T3Mois | T3Mois |
|---|---|---|---|---|---|
| Aspect macroscopique | Emulsion blanche, lisse, opaque et fluide | TA | Conforme | Conforme | Conforme |
| | | 40°C | Conforme | Conforme | Conforme |
| Observations microscopiques X400 | Emulsion gouttelettes d'huile inferieures 10$\mu$m | TA | Conforme | Conforme | Conforme |
| | | 40°C | Conforme | Conforme | Conforme |
| brookfield RV DVII Aig5vit5 Viscosité cP | | TA | 37120 | 37760 | 37200 |
| | | 40°C | 40320 | 34650 | 32080 |
| pH | 3.47 | TA | 3.36 | 3.44 | 3.37 |
| | | 40°C | 3.48 | 3.52 | 3.36 |

Tableau Ic :

| Formule A 6 | T0 | | T1Mois | T2Mois | T3Mois |
|---|---|---|---|---|---|
| Aspect macroscopique | Emulsion blanc cassé, opaque, lisse, légèrement épaisse | TA | Conforme | Conforme | Conforme |
| | | 40°C | Conforme | Conforme | Conforme |
| Observations microscopiques X400 | Emulsion gouttelettes d'huile inferieures 55$\mu$m | TA | Conforme | Conforme | Conforme |
| | | 40°C | Conforme | Conforme | Conforme |
| Viscosité cP brookfield LV DVII Aig5vit10 | 15720 | TA | 13640 | 13480 | 14000 |
| | | 40°C | 14840 | 13680 | 13640 |
| pH | 3.52 | TA | 3.43 | 3.56 | 3.80 |
| | | 40°C | 3.56 | 3.69 | 3.79 |

Tableau Id :

| Formule A 8 | T0 | | T1Mois | T3Mois |
|---|---|---|---|---|
| Aspect macroscopique | Emulsion blanche, lisse, opaque et épaisse | TA | Conforme | Conforme |
| | | 40°C | Conforme | Conforme |
| Observations microscopiques X400 | Emulsion gouttelettes d'huile inferieures 20$\mu$m | TA | Conforme | Conforme |
| | | 40°C | Conforme | Conforme |
| brookfield RV DVII Aig5vit5 Viscosité cP | 22000 | TA | 20160 | 23680 |
| | | 40°C | 23680 | 20800 |
| pH | 3.36 | TA | 3.77 | 3.44 |
| | | 40°C | 3.57 | 3.41 |

Tableau Ie :

| Formule A 9 | T0 | | T1Mois | T3Mois |
|---|---|---|---|---|
| Aspect macroscopique | Emulsion blanche, lisse, opaque et fluide | TA | Conforme | Conforme |
| | | 40°C | Conforme | Conforme |
| Observations microscopiques X400 | Emulsion gouttelettes d'huile inferieures 20μm | TA | Conforme | Conforme |
| | | 40°C | Conforme | Conforme |
| brookfield RV DVII Aig5vit5 Viscosité cP | 22240 | TA | 18400 | 20720 |
| | | 40°C | 17760 | 20880 |
| pH | 3.47 | TA | 3.36 | 3.44 |
| | | 40°C | 3.48 | 3.52 |

Tableau If :

| Formule A 10 | T0 | | T1Mois | T3Mois | T3Mois |
|---|---|---|---|---|---|
| Aspect macroscopique | Gel beige fluide, opaque, lisse | TA | Conforme | Conforme | Conforme |
| | | 40°C | Conforme | Conforme | Conforme |
| Observations microscopiques X400 | Dispersion homogène inférieur à 100μm | TA | Conforme | Conforme | Conforme |
| | | 40°C | Conforme | Conforme | Conforme |
| brookfield RV DVII Aig4vit20 Viscosité cP | 5200 | TA | 4220 | 4140 | 4160 |
| | | 40°C | 4180 | 4200 | 4140 |
| pH | 3.36 | TA | 3.86 | 3.86 | 3.89 |
| | | 40°C | 3.74 | 3.92 | 3.97 |

Les tableaux ci-dessous rassemblent les données de stabilité chimique de l'ivermectine dans ces mêmes formules intermédiaires, ainsi que dans les formules B6 et B10.

| Formule A2 | T0 | | T1Mois | T2Mois | T3Mois |
|---|---|---|---|---|---|
| % ivermectine (HPLC) | 98.1 | RT | 97.4 | 98.8 | 99.4 |
| | | 40°C | - | 92.9 | 95.8 |

| Formule A4 | T0 | | T1Mois | T2Mois | T3Mois |
|---|---|---|---|---|---|
| % ivermectine (HPLC) | 98.0 | RT | 97.1 | 98.2 | 96.1 |
| | | 40°C | - | 95.8 | 95.3 |
| Formule A6 | T0 | | T1 Mois | T2Mois | T3Mois |
| % ivermectine (HPLC) | 95.1 | RT | 95.4 | 95.3 | 93.6 |
| | | 40°C | 95.2 | 94.1 | 91.7 |

| Formule A8 | T0 | | T1Mois | T2Mois |
|---|---|---|---|---|
| % ivermectine (HPLC) | 96.3 | RT | 96.2 | 95.2 |
| | | 40°C | 96.1 | 92.9 |

| Formule A9 | T0 | | T1Mois | T2Mois |
|---|---|---|---|---|
| % ivermectine (HPLC) | 97.2 | RT | 97.4 | 96.2 |
| | | 40°C | 96.1 | 91.8 |

| Formule A10 | T0 | | T1Mois | T2Mois |
|---|---|---|---|---|
| % ivermectine (HPLC) | 97.3 | RT | 96.2 | 96.9 |
| | | 40°C | 96.1 | 97.7 |

| Formule B6 | T0 | | T1Mois | T2Mois | T3Mois |
|---|---|---|---|---|---|
| % ivermectine (HPLC) | 96 | RT | 96.9 | 97.6 | 97.6 |
| | | 40°C | - | 93.5 | 93.4 |

| Formule B10 | T0 | | T1Mois | T2Mois | T3Mois |
|---|---|---|---|---|---|
| % ivermectine (HPLC) | 97.7 | RT | 96.7 | 97.5 | 98.3 |
| | | 40°C | - | 88.5 | 93.6 |

EXEMPLE 3 : Etude comparative de mesure de l'irritation

Protocole d'étude.

L'étude est réalisée selon le protocole TG439 OCDE en vigueur pour le temps d'application court (temps de contact RHE/produit 15min). Ce protocole est adapté pour un temps d'application long (temps de contact RHE/produit 18h).

L'objectif de cette étude est d'évaluer la tolérance des supports des formules complètes et intermédiaires sur épidermes humains reconstruits (RHE, modèle Episkin) au travers de :

- l'évaluation de la réduction du MTT (viabilité cellulaire)

- la mesure de la libération d'IL-1alpha (marqueur d'irritation)

Les formules testées sont :

- Une composition intermédiaire de formulation acide: exemple A7 placebo (c'est à dire, ne contenant pas li'vermectine),

- Une composition intermédiaire de formulation basique: exemple B7,

- La formule complète composée du mélange: A7 placebo + B7 (dans un ratio 50/50 en poids),

- Une référence commerciale sous forme de crème.

Résultats de l'étude :

24

| Mélange testé | Exposition courte | Exposition longue | Conclusion |
|---|---|---|---|
| | Viabilité (%) | Viabilité (%) | Potentiel irritant |
| B7 | 89.8 | 93.3 | Non irritant |
| A7 placebo | 86.0 | 84.5 | Non irritant |
| Formule complète | 95.8 | 83.4 | Non irritant |
| Ref commerciale | 99.8 | 91.1 | Non irritant |

| Test item | Exposition courte | Exposition longue |
|---|---|---|
| | IL-1a vs contrôle | IL-1a vs contrôle |
| B7 | 1.5 | 2.0 |
| A7 placebo | 2.2 | 2.3 |
| Formule complète | 1.9 | 3.1 |
| Ref commerciale | 2.4 | 3.6 |

Les mesures du MTT selon le protocole OCDE en vigueur indiquent que toutes les formules testées sont non irritantes.

Le dosage d'IL-1a de la formule complète selon l'invention après un temps court et un temps long d'exposition montre un taux de marqueurs d'irritation plus bas qu'après application de la référence commerciale.

EXEMPLE 4 :

Il a été établi de façon empirique la teneur idéale en acide citrique, pyrophosphate de sodium et sodium dihydrogénophosphate monohydrate pour réagir avec 5% de bicarbonate de sodium. Les valeurs sont exprimées en pourcentage poids/poids par rapport au poids de chacune des deux formules intermédiaires.

| | Ratio1 | Ratio2 | Ratio3 |
|---|---|---|---|
| Bicarbonate de Sodium | 5% | 5% | 5% |
| Acide citrique | 3.5% | - | - |
| Disodium pyrophosphate | - | 12 | - |
| Sodium Dihydrogénophosphate monohydrate | | - | 7.2% |

Afin que le pH de la formule contenant l'activateur de gaz présente une compatibilité optimale avec la peau, nous avons ajouté du citrate de sodium afin de créer un tampon acide citrique/citrate de sodium.

Une partie du tampon l'acide citrique/citrate de sodium peut avantageusement être substituée par du disodium pyrophosphate et vice versa comme les teneurs citées à titre d'exemple dans le tableau I ci-dessous :

Tableau III : les valeurs sont exprimées en pourcentage poids/poids par rapport au poids de chacune des deux formules intermédiaires

| | E 1 | E2 | E 3 | E 4 | E 5 | E 6 | E7 |
|---|---|---|---|---|---|---|---|
| Bicarbonate de Sodium | 5% | 5% | 5% | 5% | 3% | 3% | 3% |
| Acide citrique | 3.5% | 1.75% | 1.4% | 0 | 2.1% | 1.05% | 0 |
| Citrate de Na | 2.7% | 1.3% | 1% | 0 | 1.6% | 1.15% | 0 |
| Disodium pyrophosphate | 0 | 6% | 7.2% | 12% | 0 | 3.6% | 7.2% |

Une partie du tampon l'acide citrique/citrate de sodium peut avantageusement être substituée par du sodium dihydrogénophosphate mono hydrate et vice versa comme les teneurs citées à titre d'exemple dans le tableau IV ci-dessous :

Tableau IV : les valeurs sont exprimées en pourcentage poids/poids par rapport au poids de chacune des deux formules intermédiaires

|  | E 1 | E8 | E9 |
|---|---|---|---|
| Bicarbonate de Sodium | 5% | 5% | 5% |
| Acide citrique | 3.5% | 1.5% | 0 |
| Citrate de Na | 2.7% | 0.5% | 0 |
| Sodium Dihydrogénophosphate monohydrate | 0 | 6.2% | 10% |

Dans un mode particulier, il a été déterminé que lorsque la quantité d'acide citrique est supérieure ou égale à 1,4, la quantité de mousse est optimale lorsque le disodium pyrophosphate est présent dans la composition selon l'équation suivante :

-

$$[C]=2.4[B]-2.4[A]/0.7$$

Lorsque :

[C] = teneur en poids en disodium pyrophosphate dans la composition intermédiaire A
[A] = teneur en poids en acide citrique monohydrate dans la composition intermédiaire A
[B] = teneur en poids en bicarbonate de sodium dans la composition intermédiaire B

L'équation ci-dessus permet ainsi de calculer les teneurs optimales entre le bicarbonate de sodium l'acide citrique et le pyrophosphate de sodium.

EXEMPLE 5 : Etude comparative de profil de libération - perméation

Le but de cette étude est d'évaluer la pénétration et la distribution de différentes formulations selon l'invention dans la peau humaine.

Protocole de l'étude :

Les formules testées dans cette étude sont appliquées sur des peaux humaines entières excisées montées sur cellule de Franz à raison de 5mg/cm$^2$ à 32°C.
Après 16 heures d'application, l'Ivermectine est dosée dans : la faction non absorbée, le stratum corneum, l'épiderme, le derme et le liquide récepteur.
Cette étude permet d'étudier l'influence de la formulation sur la libération du principe actif et sa perméation au travers de la peau. Objectif est de comparer la répartition de l'Ivermectine dans les différentes couches de la peau lors de l'application d'une formule bien connue et de l'application d'une composition sous forme de mousse chimique.
Les formules testées sont :

- Une formule de Reference sous forme de crème contenant 1% d'Ivermectine
- Une formule de mousse chimique complète à 1% d'Ivermectine composée du mélange: A4+ B9 (dans un ratio 50/50 en poids),
- Une formule de mousse chimique complète à 1 % d'Ivermectine composée du mélange: A5+ B10(dans un ratio 50/50 en poids),

Cellules de diffusion :

Les cellules de diffusion utilisées sont des cellules de diffusion statiques, sur la base de cellule de diffusion modèle de Franz, avec les caractéristiques suivantes:

- Domaine d'application = 2 cm$^2$
- Volume du compartiment de fluide récepteur = 3 ml

Le compartiment récepteur est entouré par une chemise d'eau chauffée à 37°C $\pm$ 1°C, assurant une température de 32°C $\pm$ 1°C à la surface de la peau. Le compartiment récepteur est séparé du compartiment donneur par la membrane de peau, la face épidermique étant du côté du donneur. Le compartiment Récepteur contenant un barreau d'agitation magnétique, a été rempli avec le fluide récepteur de manière à empêcher toute formation de bulles d'air. Durant le temps de diffusion, le fluide de récepteur était agité en continu afin d'assurer l'homogénéisation

Préparation des échantillons de peau :

Des peaux abdominales issues de chirurgie esthétique ont été utilisées dans cette étude. Dès l'arrivée des échantillons, l'hypoderme a été séparé de l'ensemble à l'aide d'une pince à épiler et les restes ont été doucement lavés et stockés à plat dans une feuille d'aluminium pour la congélation à -20 ° C. Le jour de l'expérimentation les échantillons de peaux ont été décongelés puis coupés en morceaux pour être compatibles avec la géométrie de la cellule de diffusion. Les échantillons de peau issus de donneurs âgé de 42, 44 et 69 ans ont été montés sur la cellule de diffusion avec du PBS en tant que fluide récepteur. L'épaisseur moyenne de la peau était de 0,89 $\pm$ 0,07 mm avec un maximum de 1,39 mm et un minimum de 0,45 mm. Epaisseurs de tous les spécimens.

Après au moins 45 min à l'équilibre avec le fluide récepteur, l'intégrité de la peau est évaluée par la mesure trans-épidermique de perte insensible en eau (TEWL). Toutes les cellules où les mesures de TEWL en dehors des critères d'acceptation sont soigneusement nettoyées et laissées à l'équilibre pendant une période prolongée avant une nouvelle mesure de la TEWL. La Valeur moyenne TEWL était de 5,51 $\pm$ 1,63 g / m$^2$ / h.

Paramètres de l'étude:

Température ambiante: 21,7 ° C
Humidité relative: 45,6%

Résultats de l'étude :

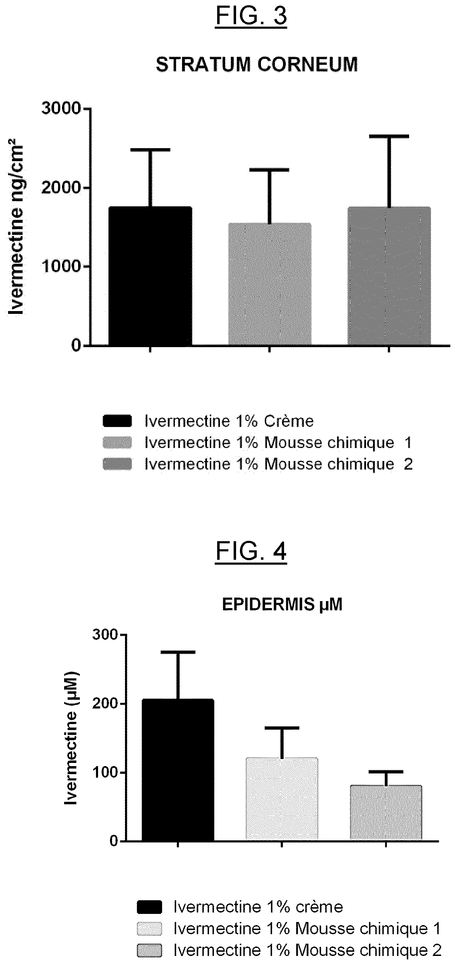

Dans le stratum corneum, il n'y avait pas de différences significatives de pénétration cutanée entre les formules mousse selon l'invention et la référence comme illustré dans la figure 3. Dans l'épiderme, les formulations de mousse chimique pénètrent moins que la référence en particulier pour la mousse 2 (selon l'exemple A5 + B10 en mélange 50:50), comme illustré dans la figure 4.

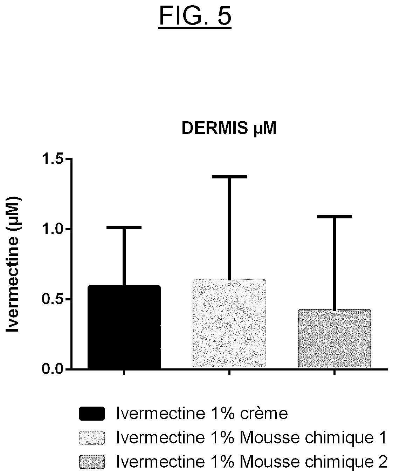

Dans le derme, aucune différence significative n'a été observée (figure 5), même si une légère différence a été observée avec la mousse chimique 2 pour laquelle des valeurs en dessous des limites de quantification (BLQ) ont été enregistrées alors que tous les échantillons du derme étaient quantifiables pour les autres formulations.

Aucun des échantillons de fluide récepteur était quantifiable quelle que soit la formulation testée; ce qui suggère une faible exposition systémique.La présente étude confirme le besoin d'obtenir une mousse qui reste en surface de la peau, afin d'agir efficacement contre le Demodex.

EXEMPLE 6 : Stabilité de la mousse (composé du mélange des formules intermédaires A et B)

Afin de démonter la remarquable stabilité de la mousse, le mélange des intermédiaires de formulation A4 et B4 dans un rapport 50/50 est réalisé dans un bêcher.

La mousse formée est photographiée à intervalles réguliers. Ces photographies représentées dans la figure 2 montrent que 30 min après le mélange, la mousse conserve le même volume qu'à T0. Ces données confirment la remarquable stabilité de la mousse selon l'invention.

**Revendications**

1. Composition auto-moussante contenant de l'ivermectine destinée à une application topique non rincée, comprenant:

- au moins une composition intermédiaire B comprenant un agent générateur de gaz et au moins un agent gélifiant et/ou agent de suspension choisi parmi les polymères d'acide acrylique, les polysaccharides, famille des aluminium magnésium silicates, la famille des amidons modifiés et la famille des carraghénanes,
- au moins une composition intermédiaire A comprenant un agent activateur de l'agent générateur de gaz et au moins un agent gélifiant et/ou agent de suspension choisi parmi les polysaccharides, la famille de aluminium magnésium silicates, la famille des amidons modifiés, la famille des carraghénanes et le PVA, et

- de l'ivermectine contenue dans la composition A, dans la composition B ou simultanément dans les deux compositions A et B.

2. Composition selon la revendication précédente, **caractérisée en ce que** l'ivermectine est contenue dans la composition intermédiaire A.

3. Composition selon l'une quelconque des revendications précédentes, **caractérisée en ce qu'**elle ne comprend pas de tensioactifs moussants, et en particulier pas de tensioactifs choisis parmi les tensioactifs anioniques, les tensioactifs cationiques, les tensioactifs amphotères et les tensioactifs non ioniques de la famille des alkylpolyglucosides, et des glucamides.

4. Composition selon l'une quelconque des revendications précédentes, **caractérisée en ce que** l'agent générateur de gaz est choisi parmi le bicarbonate de sodium, le bicarbonate de potassium, le carbonate de sodium, le carbonate de potassium et leurs mélanges, et de préférence l'agent générateur de gaz est le bicarbonate de sodium.

5. Composition selon l'une quelconque des revendications précédentes, **caractérisée en ce que** l'agent générateur de gaz est présent dans la composition intermédiaire B en une quantité allant de 1% à 10%, préférentiellement de 2% à 8% en poids, par rapport au poids de la composition intermédiaire B.

6. Composition selon l'une quelconque des revendications précédentes, **caractérisée en ce que** l'agent activateur de l'agent générateur de gaz est choisi parmi un acide, un sel de polyacide partiellement salifié, une solution tampon d'acide faible et de sa base conjuguée, et les mélanges de ces composés.

7. Composition selon la revendication précédente, **caractérisée en ce que** l'agent activateur de l'agent générateur de gaz est choisi parmi l'acide citrique, l'acide tartrique, l'acide malique, l'acide lactique, l'acide phosphorique et l'acide pyrophosphorique, et les sels de ces acides, et plus préférentiellement ledit agent activateur est choisi parmi :

- un tampon acide tartrique/sel de tartrate ;
- un tampon acide citrique/citrate de sodium seul ;
- l'acide phosphorique, le phosphate de sodium, le disodium pyrophosphate seuls ou en mélange avec un tampon acide citrique/ citrate de sodium.

8. Composition selon la revendication précédente, **caractérisée en ce que** l'agent activateur de l'agent générateur de gaz est un tampon acide citrique/ citrate de sodium seul ou en mélange avec du phosphate sodique et/ou du disodium pyrophosphate.

9. Composition sous forme de mousse, **caractérisée en ce qu'**elle résulte du mélange desdites compositions intermédiaires A et B selon l'une quelconque des revendications précédentes.

10. Composition selon l'une quelconque des revendications précédentes, **caractérisée en ce qu'**elle contient en outre un ou plusieurs actifs choisi parmi les agents émollients, les agents humectants, les agents anti-radicalaires, les agents anti-inflammatoires, les vitamines, les agents dépigmentants, les agents anti-acnéiques, les agents anti-séborrhiques, les agents anti-fongiques, les agents kératolytiques, les filtres solaires, les agents amincissants, les agents de coloration de la peau.

11. Médicament destiné à une application topique sur la peau, comprenant une composition telle que définie dans l'une quelconque des revendications 1 à 10.

12. Composition selon l'une quelconque des revendications 1 à 10, pour son utilisation dans le traitement et/ou la prévention de l'acné, de l'eczema, de la dermatite atopique, du psoriasis ou de la rosacée et de préférence pour le traitement et/ou la prévention de de la rosacée.

13. Kit ou contenant unique à plusieurs compartiments destiné à une application topique non rincée comprenant de manière séparée au moins deux compositions intermédiaires:

- une composition intermédiaire A comprenant au moins un agent activateur de l'agent générateur de gaz, telle que définie dans l'une quelconque des revendications 1 à 10; et
- une composition intermédiaire B comprenant au moins un agent générateur de gaz telle que définie dans l'une quelconque des revendications 1 à 10;
- de l'ivermectine étant contenu dans l'une au moins desdites compositions intermédiaires A et B.

**14.** Kit ou contenant unique à plusieurs compartiments selon la revendication 13, **caractérisé en ce qu'**il est conçu pour mélanger les compositions intermédiaires A et B en un rapport en poids A/B allant de 0,5 à 2, préférentiellement de 0,5 à 1,5, plus préférentiellement de 0,9 à 1,1, et encore plus préférentiellement de 1.

**15.** Procédé de préparation d'une composition sous forme de mousse destinée à une application topique non rincée contenant de l'ivermectine, dans lequel on mélange une composition intermédiaire A telle que définie dans l'une quelconque des revendications 1 à 10, avec une composition intermédiaire B telle que définie dans l'une quelconque des revendications 1 à 10, dans des proportions relatives en poids A/B allant de 0,5 à 2, préférentiellement de 0,5 à 1,5 et plus préférentiellement de 1.

**Patentansprüche**

**1.** Selbstschäumende Zusammensetzung, die Ivermectin enthält, zur topischen Anwendung ohne Ausspülen, die Folgendes umfasst:

- mindestens eine Zwischenzusammensetzung B, die ein gaserzeugendes Mittel und mindestens ein Geliermittel und/oder Suspendiermittel, das aus Acrylsäurepolymeren, Polysacchariden, der Familie der Aluminiummagnesiumsilikate, der Familie der modifizierten Stärken und der Familie der Carrageenane ausgewählt ist, umfasst,
- mindestens eine Zwischenzusammensetzung A, die ein Aktivierungsmittel für das gaserzeugende Mittel und mindestens ein Geliermittel und/oder Suspendiermittel, das aus Polysacchariden, der Familie der Aluminiummagnesiumsilikate, der Familie der modifizierten Stärken, der Familie der Carrageenane und PVA ausgewählt ist, umfasst, und
- Ivermectin, das in der Zusammensetzung A, in der Zusammensetzung B oder gleichzeitig in den beiden Zusammensetzungen A und B enthalten ist.

**2.** Zusammensetzung nach dem vorhergehenden Anspruch, **dadurch gekennzeichnet, dass** das Ivermectin in der Zwischenzusammensetzung A enthalten ist.

**3.** Zusammensetzung nach einem der vorhergehenden Ansprüche, **dadurch gekennzeichnet, dass** sie keine schäumenden Tenside und insbesondere keine Tenside, die aus anionischen Tensiden, kationischen Tensiden, amphoteren Tensiden und nichtionischen Tensiden aus der Familie der Alkylpolyglucoside und Glucamide ausgewählt sind, umfasst.

**4.** Zusammensetzung nach einem der vorhergehenden Ansprüche, **dadurch gekennzeichnet, dass** das gaserzeugende Mittel aus Natriumbicarbonat, Kaliumbicarbonat, Natriumcarbonat, Kaliumcarbonat und Mischungen davon ausgewählt ist und es sich vorzugsweise bei dem gaserzeugenden Mittel um Natriumbicarbonat handelt.

**5.** Zusammensetzung nach einem der vorhergehenden Ansprüche, **dadurch gekennzeichnet, dass** das gaserzeugende Mittel in der Zwischenzusammensetzung B in einer Menge von 1 bis 10 Gew.-%, bevorzugt von 2 bis 8 Gew.-%, bezogen auf das Gewicht der Zwischenzusammensetzung B, vorliegt.

**6.** Zusammensetzung nach einem der vorhergehenden Ansprüche, **dadurch gekennzeichnet, dass** das Aktivierungsmittel für das gaserzeugende Mittel aus einer Säure, einem partiell versalzten Polysäuresalz, einer Pufferlösung einer schwachen Säure und ihrer konjugierten Base und Mischungen dieser Verbindungen ausgewählt ist.

**7.** Zusammensetzung nach dem vorhergehenden Anspruch, **dadurch gekennzeichnet, dass** das Aktivierungsmittel für das gaserzeugende Mittel aus Citronensäure, Weinsäure, Äpfelsäure, Milchsäure, Phosphorsäure und Pyrophosphorsäure und den Salzen dieser Säuren ausgewählt ist und weiter bevorzugt das Aktivierungsmittel aus

- einem Weinsäure/Tartratsalz-Puffer;
- einem Citronensäure/Natriumcitrat-Puffer allein;
- Phosphorsäure, Natriumphosphat, Dinatriumpyrophosphat allein oder im Gemisch mit einem Citronensäure/Natriumcitrat-Puffer

ausgewählt ist.

8.  Zusammensetzung nach dem vorhergehenden Anspruch, **dadurch gekennzeichnet, dass** es sich bei dem Aktivierungsmittel für das gaserzeugende Mittel um einen Citronensäure/Natriumcitrat-Puffer allein oder im Gemisch mit Natriumphosphat und/oder Dinatriumpyrophosphat handelt.

9.  Zusammensetzung in Schaumform, **dadurch gekennzeichnet, dass** sie aus dem Mischen der Zwischenzusammensetzungen A und B nach einem der vorhergehenden Ansprüche resultiert.

10. Zusammensetzung nach einem der vorhergehenden Ansprüche, **dadurch gekennzeichnet, dass** sie außerdem einen oder mehrere Wirkstoffe, die aus Emollientien, Feuchthaltemitteln, Radikalfängern, entzündungshemmenden Mitteln, Vitaminen, Depigmentierungsmitteln, Antiaknemitteln, antiseborrhoischen Mitteln, Fungiziden, Keratolytika, Sonnenschutzmitteln, Schlankmachern und Hautfärbemitteln ausgewählt sind, enthält.

11. Medikament zur topischen Anwendung auf der Haut, umfassend eine Zusammensetzung gemäß einem der Ansprüche 1 bis 10.

12. Zusammensetzung nach einem der Ansprüche 1 bis 10 zur Verwendung bei der Behandlung und/oder Prävention von Akne, Ekzem, atopischer Dermatitis, Psoriasis oder Rosacea und vorzugsweise für die Behandlung und/oder Prävention von Rosacea.

13. Kit oder einzelner Behälter mit mehreren Fächern zur topischen Anwendung ohne Ausspülen, das/der in getrennter Form mindestens zwei Zwischenzusammensetzungen umfasst:

    - eine Zwischenzusammensetzung A, die mindestens ein Aktivierungsmittel für das gaserzeugende Mittel umfasst, gemäß einem der Ansprüche 1 bis 10 und
    - eine Zwischenzusammensetzung B, die mindestens ein gaserzeugendes Mittel umfasst, gemäß einem der Ansprüche 1 bis 10,
    - Ivermectin, das in mindestens einer der Zwischenzusammensetzungen A und B enthalten ist.

14. Kit oder einzelner Behälter mit mehreren Fächern nach Anspruch 13, **dadurch gekennzeichnet, dass** es/er dafür ausgelegt ist, die Zwischenzusammensetzungen A und B in einem Gewichtsverhältnis A/B im Bereich von 0,5 bis 2, bevorzugt von 0,5 bis 1,5, weiter bevorzugt von 0,9 bis 1,1 und noch weiter bevorzugt von 1 zu mischen.

15. Verfahren zur Herstellung einer Zusammensetzung in Schaumform zur topischen Anwendung ohne Ausspülen, die Ivermectin enthält, bei dem man eine Zwischenzusammensetzung A gemäß einem der Ansprüche 1 bis 10 mit einer Zwischenzusammensetzung B gemäß einem der Ansprüche 1 bis 10 in relativen Gewichtsanteilen A/B im Bereich von 0,5 bis 2, weiter bevorzugt von 0,5 bis 1,5 und noch weiter bevorzugt von 1 mischt.

**Claims**

1.  A self-foaming composition containing ivermectin, intended for leave-on topical application, comprising:

    - at least one intermediate composition B comprising a gas-generating agent and at least one gelling agent and/or suspending agent chosen from acrylic acid polymers, polysaccharides, the family of magnesium aluminum silicates, the family of modified starches and the family of carrageenans,
    - at least one intermediate composition A comprising an agent for activating the gas-generating agent and at least one gelling agent and/or suspending agent chosen from polysaccharides, the family of magnesium aluminum silicates, the family of modified starches, the family of carrageenans and PVA, and
    - ivermectin contained in composition A, in composition B or simultaneously in the two compositions A and B.

2.  The composition as claimed in the preceding claim, **characterized in that** ivermectin is contained in the intermediate composition A.

3.  The composition as claimed in either of the preceding claims, **characterized in that** it does not comprise any foaming surfactants, and in particular no surfactants chosen from anionic surfactants, cationic surfactants, amphoteric surfactants and nonionic surfactants of the family of alkylpolyglucosides and glucamides.

4. The composition as claimed in any one of the preceding claims, **characterized in that** the gas-generating agent is chosen from sodium bicarbonate, potassium bicarbonate, sodium carbonate and potassium carbonate, and mixtures thereof, and preferably the gas-generating agent is sodium bicarbonate.

5. The composition as claimed in any one of the preceding claims, **characterized in that** the gas-generating agent is present in the intermediate composition B in an amount ranging from 1% to 10% by weight and preferentially from 2% to 8% by weight, relative to the weight of the intermediate composition B.

6. The composition as claimed in any one of the preceding claims, **characterized in that** the agent for activating the gas-generating agent is chosen from an acid, a partially salified polyacid salt, a buffer solution of a weak acid and of its conjugate base, and mixtures of these compounds.

7. The composition as claimed in the preceding claim, **characterized in that** the agent for activating the gas-generating agent is chosen from citric acid, tartaric acid, malic acid, lactic acid, phosphoric acid and pyrophosphoric acid, and the salts of these acids, and more preferentially said activating agent is chosen from:

- a tartaric acid/tartrate salt buffer;
- a citric acid/sodium citrate buffer alone;
- phosphoric acid, sodium phosphate, disodium pyrophosphate, which are alone or as a mixture with a citric acid/sodium citrate buffer.

8. The composition as claimed in the preceding claim, **characterized in that** the agent for activating the gas-generating agent is a citric acid/sodium citrate buffer, alone or as a mixture with sodium phosphate and/or disodium pyrophosphate.

9. A composition in foam form, **characterized in that** it results from the mixing of said intermediate compositions A and B as claimed in any one of the preceding claims.

10. The composition as claimed in any one of the preceding claims, **characterized in that** it also contains one or more active agents chosen from emollients, humectants, free-radical scavengers, anti-inflammatory agents, vitamins, depigmenting agents, antiacne agents, antiseborrheic agents, antifungal agents, keratolytic agents, sunscreens, slimming agents and skin-coloring agents.

11. A medicament intended for topical application to the skin, comprising a composition as defined in any one of claims 1 to 10.

12. The composition as claimed in any one of claims 1 to 10, for its use in the treatment and/or prevention of acne, eczema, atopic dermatitis, psoriasis or rosacea and preferably for treating and/or preventing rosacea.

13. A kit or single multi-compartment container intended for leave-on topical application separately comprising at least two intermediate compositions:

- an intermediate composition A comprising at least one agent for activating the gas-generating agent, as defined in any one of claims 1 to 10; and
- an intermediate composition B comprising at least one gas-generating agent as defined in any one of claims 1 to 10;
- ivermectin being contained in at least one of said intermediate compositions A and B.

14. The kit or single multi-compartment container as claimed in claim 13, **characterized in that** it is designed for mixing the intermediate compositions A and B in an A/B weight ratio ranging from 0.5 to 2, preferentially from 0.5 to 1.5, more preferentially from 0.9 to 1.1 and even more preferentially 1.

15. A process for preparing a composition in foam form intended for leave-on topical application, containing ivermectin, in which an intermediate composition A as defined in any one of claims 1 to 10 is mixed with an intermediate composition B as defined in any one of claims 1 to 10, in relative weight proportions A/B ranging from 0.5 to 2, preferentially from 0.5 to 1.5 and more preferentially 1.

# FIG. 1

| A4 + B4 (50/50) avant mélange | A4+B4 (50/50) après mélange |

# FIG. 2

| | |
|---|---|
| A4+B4 (50/50) après mélange | |
| A4+B4 (50/50) 15 min après le mélange | |
| A4+B4 (50/50) 30 min après le mélange | |

## FIG. 3

### STRATUM CORNEUM

■ Ivermectine 1% Crème
▨ Ivermectine 1% Mousse chimique 1
▨ Ivermectine 1% Mousse chimique 2

## FIG. 4

### EPIDERMIS µM

■ Ivermectine 1% crème
□ Ivermectine 1% Mousse chimique 1
▨ Ivermectine 1% Mousse chimique 2

# FIG. 5

DERMIS µM

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 6133310 A
- US 5952372 A

**Littérature non-brevet citée dans la description**

- The extra pharmacopoeia. Pharmaceutical Press, 1993
- **CAMPBELL, W.C. et al.** Ivermectin: a patent new antiparasitic agent. *Science,* 1983, vol. 221, 823-828